# EUROPEAN PATENT APPLICATION

(11) **EP 3 885 437 A1**
(43) Date of publication of application: **29.09.2021**
(21) Application number: 20166145.1
(22) Date of filing: 27.03.2020
(51) Int. Cl.: C12N 15/10, C40B 40/06, C40B 50/08, C07H 19/23, C07H 21/00

(54) **DNA-ENCODED CHEMICAL LIBRARY, USE THEREOF, AND METHOD TO SYNTHESIZE THE LIBRARY**

(71) Applicant: Technische Universität Dortmund, 44227 Dortmund (DE)
(72) Inventor: BRUNSCHWEIGER, Andreas, 44265 Dortmund (DE); KLIKA SKOPIC, Mateja, 44388 Dortmund (DE); POTOWSKI, Marco, 46244 Bottrop (DE); KUNIG, Verena Barbara Katharina, 44227 Dortmund (DE)
(74) Representative: Viering, Jentschura & Partner mbB Patent- und Rechtsanwälte

(57) **Abstract**

The present invention relates to a compound library comprising a plurality of conjugate molecules, wherein said conjugates comprise a small organic molecule covalently coupled to a nucleic acid moiety, wherein the nucleic acid moiety comprises or consists of 7-deazapurines and/or 7-deaza-8-azapurines, and, optionally, modified and/or unmodified pyrimidine nucleotides. Further, the present invention relates to the use of said library for screening compounds binding to a target molecule and methods of synthesizing said library.

## Description

### FIELD OF THE INVENTION

The present invention lies in the field of medicinal chemistry and chemical biology and relates to a compound library comprising a plurality of conjugate molecules, wherein said conjugates comprise a small organic molecule covalently coupled to a nucleic acid moiety, wherein the nucleic acid moiety comprises or consists of 7-deazapurines and/or 7-deaza-8-azapurines, and, optionally, modified and/or unmodified pyrimidine nucleotides. Further, the present invention relates to the use of said library for screening compounds binding to a target molecule and methods of synthesizing said library.

### BACKGROUND OF THE INVENTION

DNA-encoded chemical libraries (DELs) represent a technology for drug discovery. DELs feature the display of individual small organic chemical moieties on DNA tags that serve as amplifiable identification barcodes. The DNA-tag allows the simultaneous screening of very large compound collections (up to billions of molecules), because the DNA-tag of the relevant hit compounds can be identified and quantified by PCR-amplification and sequencing. They combine an ultra-high throughput compound screening approach with unbiased affinity-based compound identification. The most common approach to DNA-encoded libraries is the combinatorial mix-and-split synthesis strategy that combines preparative organic synthesis and encoding steps performed in an iterative manner.

Library design is enabled by a combination of starting materials and synthesis methodology. However, three factors pose serious limitations upon design of encoded libraries:
- the chemical lability of DNA, e.g. DNA integrity can be compromised by depurination under many reaction conditions required for small molecule synthesis (see lit:.M. Potowski, F. Losch, E. Wunnemann, J. K. Dahmen, S. Chines, A. Brunschweiger, Screening of metal ions and organocatalysts on solid support-coupled DNA oligonucleotides guides design of DNA-encoded reactions. Chem. Sci. 2019 10, 10481-10492.);
- the need to perform reactions in aqueous co-solvents; and
- the need to use reactions with fast kinetics due to high dilution of DNA-coupled substrates.

A prerequisite for the library synthesis is the compatibility of DNA with the synthesis methodology used for synthesis of the organic chemical moieties. The main DNA degradation reaction is caused by depurination, i.e. the cleavage of purine bases from the DNA oligomer. DNA can be depurinated under acidic conditions and upon incubation with Lewis acids under forcing reaction conditions such as elevated temperature. (see lit:.M. Potowski, F. Losch, E. Wunnemann, J. K. Dahmen, S. Chines, A. Brunschweiger, Screening of metal ions and organocatalysts on solid support-coupled DNA oligonucleotides guides design of DNA-encoded reactions. Chem. Sci. 2019 10, 10481-10492.)

Consequently, any reaction methodology meeting the compatibility requirement is very limited. A current challenge for DEL synthesis research is the development of novel synthetic schemes that furnish in DNA-compatible manner DNA-conjugates of small and geometrically defined (rigid) scaffolds that serve as starting points for subsequent combinatorial library synthesis. The synthesis of such molecules would result in molecules having a drug-like structure. These drug-like structures include, but are not limited to heterocyclic structures.

To date, the use of DNA that was composed of the nucleobases thymine, cytidine and 7-deazaadenine has been shown for barcoding of one-bead-one-compound peptide libraries (M. C. Needels, D. G. Jones, E. H. Tate, G. L. Heinkel, L. M. Kochersberger, W. J. Dower, R. W. Barrett, M. A. Gallop, Proc. Natl. Acad. Sci. USA, 1993, 90, 10700-10704). This technology required elongation of the code by chemical phosphoramidate DNA synthesis. It was previously demonstrated that the use of 7-deaza-8-azapurines for PCR amplification of 7-deaza-8-azapurine-containing DNA template strands was not possible (E. Eremeeva, et al., The 5-chlorouracil:7-deazaadenine base pair as an alternative to the dT:dA base pair. Org. Biomol. Chem., 2017, 15, 168).

### SUMMARY OF THE INVENTION

Surprisingly, the present inventors found a synthesis strategy that enables the synthesis of several (hetero)cyclic structures from different starting materials attached to a specific DNA sequence by various catalytic methods. Fundamental to the present invention is the observation that DNA sequences that comprise 7-deazapurines and/or 7-deaza-8-azapurines as purine bases and, optionally, modified and/or unmodified pyrimidine nucleotides are tolerant to a broad spectrum of reaction conditions, such as the use of strong acids or high concentrations of transition metals as catalysts; whereas, as experimentally proven, DNA molecules comprising natural purines adenine and guanine are degraded under the same reaction conditions by depurination and subsequent strand fragmentation of the resulting abasic sites. (M. Potowski, F. Losch, E. Wunnemann, J. K. Dahmen, S. Chines, A. Brunschweiger, Screening of metal ions and organocatalysts on solid support-coupled DNA oligonucleotides guides design of DNA-encoded reactions. Chem. Sci. 2019 10, 10481-10492.) The possibility to use a broad spectrum of reaction conditions allows for the synthesis of several heterocyclic structures attached to DNA sequences. In turn, this possibility to apply a broader spectrum of chemical reaction conditions results in the synthesis of a broad range of drug-like small molecule structures.

DNA-barcoding of compounds may require two enzymatic steps, preferably 5'-terminal phosphorylation of a first code sequence with polynucleotide kinase and ligation with a ligase, such as T4 ligase, to a second or further code sequence. The readout of the codes by sequencing requires prior amplification of the DNA strand with a polymerase, such as Taq polymerase. These enzymatic steps must be compatible with the chemical composition of the DNA. Surprisingly, the present inventors found that DNA sequences that comprise or consist of 7-deazapurines and/or 7-deaza-8-azapurines, and, optionally, modified and/or unmodified pyrimidine nucleotides can be phosphorylated at the 5'-terminus of the DNA sequence by a polynucleotide kinase. Surprisingly, DNA sequences containing the aforementioned chemical modifications may also be ligated by a DNA ligase, such as T4 DNA ligase. Surprisingly, DNA sequences ligated from chemically modified DNA fragments containing the aforementioned modified purine nucleobases can be read with high fidelity by Taq polymerase as experimentally demonstrated by Sanger sequencing for the first time (see Examples).

An encoding scheme may enable initiation of DNA-encoded library synthesis with controlled pore glass-coupled DNA strands (M. Potowski, V. Kunig, F. Losch, A. Brunschweiger, Synthesis of DNA-coupled isoquinolones and pyrrolidines by solid phase ytterbium- and silver-mediated imine chemistry. Med. Chem. Commun. 2019, 10, 1082-1093).

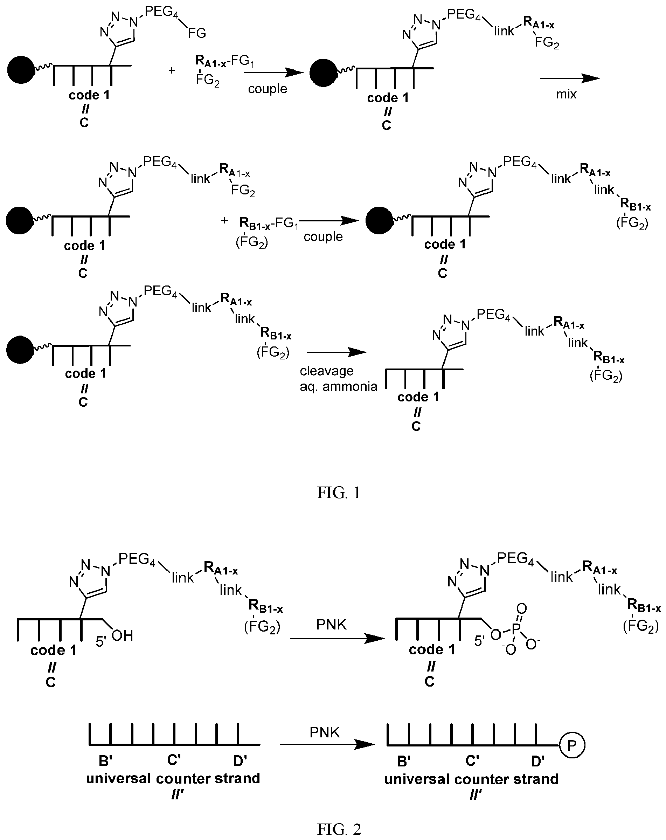

Herein, the application of this barcoding strategy for the use of chemically modified DNA strands and for ligating a pool of chemically stabilized DNA sequences to a PCR primer and to a second code is shown for the first time. A pool of chemically stabilized DNA oligonucleotides of different sequences can be annealed to a DNA oligonucleotide consisting of terminal complementary partial sequences and a degenerate middle sequence comprising inosine and stable abasic sites (see Figures 2-4; Tables 2-5, 6-8).

In a first aspect, the present invention therefore relates to a compound library comprising a plurality of conjugate molecules, wherein said conjugates comprise a small organic molecule covalently coupled to a nucleic acid moiety, wherein the nucleic acid moiety comprises or consists of 7-deazapurines and/or 7-deaza-8-azapurines, and, optionally, modified and/or unmodified pyrimidine nucleotides.

In various embodiments, the 7-deazapurines and/or 7-deaza-8-azapurines are selected from 7-deazaadenosine, 7-deaza-guanosine, 7-deaza-8-azaadenosine, 7-deaza-8-azaguanosine, or combinations thereof, preferably 7-deaza-8-azaadenosine, 7-deaza-8-azaguanosine, or combinations thereof.

In various embodiments, the 7-deazapurines and/or 7-deaza-8-azapurines comprise 7-aza- or 7-deaza-8-aza-modified inosine, 7-aza- or 7-deaza-8-aza-modified N⁶-methyladenosine, 7-aza- or 7-deaza-8-aza-modified xanthosine, or combinations thereof

In various embodiments, the plurality of conjugate molecules in the compound library comprises at least ten (different) molecules, preferably at least 15, at least 20, at least 30, at least 40 or at least 50 different molecules, more preferably at least 60, at least 70, at least 80, at least 90 or at least 100 different molecules. The molecules differ in the small organic moiety and, since each small organic moiety is identified by a specific nucleic acid sequence, also in their nucleic acid moiety.

In various embodiments, the nucleic acid moiety consists of at least 2, preferably at least 3, at least 4, at least 5, at least 6, at least 7, at least 8, at least 10 or more nucleotides. The upper limit is typically about 150 nucleotides in size, preferably up to 120 or up to 100.In various embodiments the nucleic acid moiety may comprise 60 to 90 nucleotides, such as 70 to 90 or 75 to 85. The nucleic acid moiety may comprise a code sequence that is used to identify the small organic moiety and additionally functional sequences needed for its detection, for example primer binding sites and the like.

In various embodiments, the nucleic acid moiety is RNA, DNA or a mixture thereof. In more preferred embodiments, the nucleic acid moiety is DNA, i.e. the sugar moiety is deoxyribose. The nucleic acid moiety may be single-stranded or double-stranded. In various embodiments, it is single-stranded. In various alternative embodiments, it is double-stranded.

In various embodiments, the nucleic acid moiety and the organic molecule are linked by an amide bond.

In further embodiments, the present invention relates to a compound library wherein the conjugates differ from each other by comprising different nucleic acid moieties and/or different small organic molecules. In various embodiments, each of the different small organic molecules is identifiable by a unique nucleic acid moiety that differs, in its sequence, from those coupled to other small organic molecules. This unique part of each nucleic acid moiety is also referred to herein as "code sequence". The remainder of the nucleic acid moiety that provides the desired functionality, such as amplification primer binding sites, ligation sites, etc. may be the same for all nucleic acid moiety of a given population of conjugates, as long as the code sequence is still unique for each small organic moiety.

In various embodiments, the conjugate molecules of the library further comprise a linker portion between the nucleic acid moiety and the organic moiety molecule, this linker portion may be an polyoxyalkylene group, for example polyethylene glycol (PEG), an alkylene group, such as a methylene, ethylene or propylene group, or any other suitable linker group.

In various embodiments, the organic molecule consists of 2 or more carbon atoms, such as 3, 4, 5 or more carbon atoms, preferably up to 50 carbon atoms, more preferably up to 30 carbon atoms. It may comprise heteroatoms, for example selected from O, N, and S. In various embodiments, it may be a cyclic molecule, for example a heterocyclic molecule comprising one or more heteroatoms, for example selected from N, O and S. A more detailed definition of organic molecule is provided below.

In various embodiments, the organic molecule has a molecular weight of at most 900 daltons, preferably at most 700 daltons, and more preferably at most 500 daltons.

In various embodiments, the modified or unmodified pyrimidine nucleotides are selected from the group consisting of thymidine, cytidine, uridine, 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, 1-methylpseudouridine, 3-methylcytidine, 5-methylcytidine, 5-methylaminomethyluridine, 5-methoxyaminomethyl-2-thiouridine, 5-methoxycarbonylmethyl-2-thiouridine, 5-methoxycarbonylmethyluridine, 5-methoxyuridine, uridine-5-oxyacetic acid-methylester, uridine-5-oxyacetic acid, pseudouridine, 2-thiocytidine, 5-methyl-2-thiouridine, 2-thiouridine, 4-thiouridine, 5-methyluridine, 2'-O-alkyluridine, 2'-O-alkylthymidine, 2'-O-alkylcytidine and 3-(3-amino-3-carboxy-propyl)uridine. Also encompassed are combinations thereof.

In a further aspect, the present invention relates to a method for synthesizing a compound library, wherein said library comprises a plurality of conjugate molecules, wherein said conjugates comprise a small organic molecule covalently coupled to a nucleic acid moiety, the synthesis of each conjugate molecule comprises: (1) reacting a nucleic acid consisting of 7-deazapurines and/or 7-deaza-8-azapurines and, optionally, modified and/or unmodified pyrimidine nucleotides (also referred to as "first code sequence") with an organic molecule under conditions that allow the conjugation of said molecules; optionally, (2) subjecting the conjugate obtained in step (1) to reaction conditions that allow modification of the small organic molecule (without affecting the nucleic acid moiety); and, optionally, (3) elongating the nucleic acid moiety of the conjugate obtained in step (2) by adding a further nucleic acid sequence (also referred to as "second or further code sequence"). In step (1) of said method, each organic molecule is conjugated to a unique nucleic acid molecule that allows its identification. To avoid that the nucleic acid moiety is affected by the reaction in step (2), the nucleic acid moiety in step (1) consists of 7-deazapurines and/or 7-deaza-8-azapurines, and, optionally, modified and/or unmodified pyrimidine nucleotides, i.e. does not contain unmodified purine bases. The nucleic acid sequence added in step (3) can comprise modified and/or unmodified purine and pyrimidine nucleotides, as the reaction in step (2) is already completed. In various embodiments, it is also possible to repeat steps (2) and (3) multiple times. This allows synthesis of more complex organic molecules. Depending on the type of subsequent reaction the conjugate is subjected to after the first step (3), this requires that the elongation in step (3) is carried out using a suitable nucleic acid sequence, e.g. a nucleic acid sequence that consists of 7-deazapurines and/or 7-deaza-8-azapurines, and, optionally, modified and/or unmodified pyrimidine nucleotides, and only after the final reaction step (2) is completed, the elongation may be carried out with unmodified purine and, optionally pyrimidine bases. The nucleic acid sequences referred to in this context are the code sequences that serve to identify the coupled organic moiety. It is however contemplated in various embodiments that in addition to adding a code sequence that is used for identification of the organic moiety present in the conjugate, additional nucleotide sequence elements are added that, for example, provide for detectability of the conjugate, such as primer binding sites and the like.

In various embodiments of the method, the conjugate molecule obtained in step (1) is further used in a reaction comprising a) a strong acid, preferably an acid having a pKₐ of less than 3; and/or b) the presence of a metal catalyst selected from the group consisting of copper, silver, gold, ruthenium, iron, osmium, cobalt, rhodium, iridium, nickel, palladium, platinum, ytterbium, and derivatives thereof, preferably copper, silver, gold, ruthenium, and derivatives thereof. This reaction may serve the purpose to convert the small organic molecule, the core structure/scaffold, to the desired drug candidate molecule. Due to the specific nature of the nucleic acid identifier sequence used in the conjugate, these are able to withstand these reaction conditions without adversely influencing nucleic acid structural integrity. This is in various embodiments the reaction generally referred to in step (2) above.

In another aspect, the present invention relates to the use of a compound library of the present invention for screening a compound capable of binding to a target molecule. The compound may be screened based on its affinity for the target molecule, depending on reaction conditions for binding.

In various embodiments, the target molecule is a protein.

It is understood that all combinations of the above disclosed embodiments are also intended to fall within the scope of the present invention.

### BRIEF DESCRIPTION OF THE DRAWINGS

The invention will be better understood with reference to the detailed description when considered in conjunction with the non-limiting examples and the accompanying drawings.
FIG. 1 shows the synthesis of target molecules on controlled pore glass-coupled DNA barcodes (two or three-cycle library). The designation of the sequence code 1 may be found in Tables 2-4, and code 1 identifies building block R_{A1-x}; FG is Functional group, and CPG is Controlled pore glass; the cleavage occurs with aqueous ammonia or aqueous methylamine.
**FIG. 2** shows the enzymatic phosphorylation of chemically modified code 1 DNA sequences ***II*** and degenerate counter strand ***II*'** using a polynucleotide kinase (PNK).
F**IG. 3** illustrates a two-cycle encoding strategy for short single-stranded DNA conjugates. The designation of the sequences may be found in Tables 2-4; R is PEG(4)-amine, and S is triethylene glycol spacer. In the final step the organic moiety is not depicted but is still present.
**FIG. 4** shows a hairpin (three cycle) encoding strategy for short single-stranded DNA conjugates. The designation of the sequences may be found in Tables 3-4. In the final step the organic moiety is no longer depicted but is still present.
**FIG. 5** depicts nucleosides used for barcoding of compounds. Sequence ***II*** is composed of chemically modified nucleosides; sequence ***II*'** is composed of natural nucleosides and a degenerate region composed of inosine and stable abasic building blocks.
**FIG. 6** shows an embodiment of a design of a final DNA-tagged compound and a PCR amplicon thereof.
**FIG. 7** represents a synthesis of *N*⁶-Benzoyl-2'-deoxy-5'-O-DMT-7-deaza-2'-deoxyadenosine 3'-CE phosphoramidite.

### DETAILED DESCRIPTION OF THE INVENTION

The present inventors surprisingly found that 7-deazapurines and/or 7-deaza-8-azapurines are not degraded under conditions commonly used for the synthesis of heterocyclic chemical compounds (for example reactions involving the use of strong acids or metal catalysts, such as copper, silver, gold, ruthenium, iron, osmium, cobalt, rhodium, iridium, nickel, palladium, platinum, ytterbium, and derivatives thereof), which is contrasted to most purine nucleotides that are degraded under the same conditions. Based on these results, the present inventors concluded that chemical compounds being labeled with DNA can be reacted with a broad spectrum of chemical structures, including but not limited to those necessary for forming heterocycles, if the DNA-label consists of 7-deazapurines and/or 7-deaza-8-azapurines (for example instead of unmodified purine bases) and optionally modified and/or unmodified pyrimidine nucleotides. If the reaction comprises multiple steps, after each step the conjugate may be further modified by adding a nucleic acid sequence by elongation of the existing sequence that serves to identify the reaction steps already carried out and thus identify the organic molecule part of the conjugate at each step of the reaction process. Said nucleic acid sequence added between those steps is typically of the same type as the one used for the first labeling step, as it needs to withstand the reaction conditions. At a later stage of the reaction, for example after the organic moiety of the compound has been reacted to its final chemical structure, it is also possible to elongate the DNA-label with further nucleotides including additional purine and/or pyrimidine nucleotides, with these additional nucleotides including the naturally occurring non-modified nucleotides. Specifically, the nucleic acid moiety (DNA-label) may be elongated after each reaction step/cycle that changes the organic moiety with additional DNA barcodes that comprise or consist of pyrimidine nucleotides; 7-deazapurines and/or 7-deaza-8-azapurines; native DNA nucleotides, or combinations thereof by an enzymatic ligation step, with the selection of the nucleotides used being dependent on the following reaction steps, if any, to allow the synthesis of the conjugate molecule. In all these elongation steps, the DNA barcodes may be selected such that they identify the organic moiety after the reaction. The elongated DNA label thus allows determination of the starting organic moiety (before the reaction by virtue of the original DNA label sequence) and the organic moiety after it has been subj ected to a specific type of reaction (by virtue of the elongated DNA label sequence, in particular the added sequence).

In various embodiments, the nucleic acid moiety can comprise, in addition to the nucleotide sequence stretches used for identification of the small organic moiety, additional nucleic acid sequences that are not used for identification purposes but rather to allow processing of the conjugate, such as immobilization, further conjugation to other nucleic acid molecules, amplification or detection. These nucleic acid sequences may be added in an elongation step as defined herein, with "elongation step" generally referring to any type of reaction that adds nucleotides to the first code sequence, i.e. the nucleotide sequence added to the small organic moiety used as a starting scaffold/educt for its identification. Typically, these are added after the reaction of the organic moiety is completed to avoid limitation as to their sequence.

Thus, in a first aspect, the present invention relates to a compound library comprising a plurality of conjugate molecules, wherein said conjugates comprise a small organic molecule covalently coupled to a nucleic acid moiety, wherein the nucleic acid moiety comprises or consists of 7-deazapurines and/or 7-deaza-8-azapurines, and, optionally, modified and/or unmodified pyrimidine nucleotides. Each conjugate molecule has a different organic molecule from the other conjugate molecules, i.e. the pairs of organic molecule and nucleic acid moiety are unique in that each specific organic molecule is identified by one specific nucleic acid moiety that differs in sequence from other nucleic acid moieties used for conjugation to other organic molecules. The difference in the nucleic acid sequence is, for example, limited to the so-called code sequence which may comprise or consist of a first and a second and, optionally, further code sequence, while other parts of the nucleic acid moiety may be similar in that they provide for a functionality desired for all conjugates, such as the ability to allow their amplification, etc. However, in various embodiments, also these sequences used for functional purposes may differ between the different conjugates, for example, so that discrimination can already be achieved at the amplification stage.

The term "compound", as used herein, relates to a substance formed when two or more chemical elements are chemically bonded together. A typical type of chemical bond in the compounds of the present invention is a covalent bond. However, atoms within the compound may also be bound to each other by ionic bonds, metallic bonds and/or coordinate covalent bonds. "Compounds" refer generally to molecules potentially capable of structural interactions with extracellular or cellular constituents through covalent or non-covalent interactions, such as, for example, through hydrogen bonds, ionic bonds, van der Waals attractions, or hydrophobic interactions. For example, compounds will most typically include molecules with moieties necessary for structural interaction with proteins, glycoproteins, and/or other macromolecules. "Compound library", as used herein, refers to any collection of agents/compounds that includes a plurality of molecular structures. Compound libraries can include, for example, combinatorial chemical libraries, natural products libraries, and peptide and cyclized peptide libraries. Compound libraries of the present invention contain compounds that comprise a small organic molecule covalently coupled to a nucleic acid moiety. Such libraries are known as DNA-encoded chemical libraries (DELs).

"Barcode" and "barcoding" and "coding" may be used interchangeably and are defined herein to mean the nucleic acid moiety attached to the organic molecule. The nucleic acid moiety acts as a barcode to unambiguously identify each of the conjugates in the respective binding assays. These nucleic acid sequences having barcoding properties are also referred to as "code sequences" herein.

"Plurality", as used herein, is defined as two or more than two, in particular 2, 3, 4, 5, 6, 7, 8, 9, 10 or more, more preferably 20 or more, 50 or more, 100 or more, 500 or more, 1000 or more, 1500 or more, 3000 or more, 5000 or more, 10000 or more or 50000 or more. In various embodiments, the library of the present invention comprises at least 10 (different) molecules, preferably at least 20, at least 30, at least 40 or at least 50 different molecules, more preferably at least 100 different molecules. The term "plurality" as used herein refers to the type of molecule, not the number of molecules. A plurality of conjugates thus means that a multitude of conjugate molecules is present that differ in their organic moiety and their corresponding nucleic acid moiety independent on whether each type of these molecules is present in multiple copies or only a single copy. Typically, a library as defined herein comprises a plurality of different conjugates, with each type of conjugate being represented by a high number of identical molecules.

The term "conjugate molecule", as used herein, refers to a compound comprising two or more molecules (e.g., organic molecules or nucleic acid molecules) that are chemically linked. The two or more molecules are chemically linked using any suitable chemical bond (e.g., covalent bond). Suitable chemical bonds are well known in the art and include but are not limited to amide bonds, disulfide bonds, thioester bonds, and ester bonds. According to the invention, the conjugates comprise a small organic molecule covalently coupled to a nucleic acid moiety. In various embodiments, the nucleic acid moiety and the organic molecule are linked by an amide bond. The linkage may be direct or via a linker moiety, such as those described herein.

The term "organic molecule" refers to carbon-based chemicals, including small organic molecules and macromolecules, as well as derivatives and analogues thereof. The organic molecules may display biological activity such as, but not limited to pharmaceutical, antibiotic, pesticidical, herbicidical, or fungicidical activity. Preferably, the organic molecule, in particular the final organic molecule after reaction of the conjugate, contains a heterocyclic structure. The term "heterocyclic," as used herein, means an aromatic or non-aromatic saturated mono-, bi-, or tricyclic ring system having 2 to 14 ring carbon atoms, containing 1-8 ring heteroatom or heteroatom groups, for example chosen from N, NH, N-(CO)-C₁₋₆-alkyl, NC₁₋₆-alkyl, O, SO, SO₂, S, P, or PO₄⁻³ alone or in combination. Bi- and tricyclic heterocyclic groups are fused at 2 or 4 points or joined at one point via a bond or a heteroatom linker (O, S, NH, or N(C₁-C₆ alkyl). The mono-, bi-, or tricyclic heterocyclic group can be optionally substituted on the ring by replacing an available hydrogen on the ring by one or more substituents which may be the same or different, each being independently selected from any suitable group, including but not limited to C1-10 alkyl, alkoxy, alkenyl, alkenyloxy, alkynyl or alkynyloxy, 5- to 14-membered aryl, heteroaryl, or cycloaliphatic, halogen, nitro, cyano, hydroxy, amino, and carboxy. In a non-limiting embodiment, there are no adjacent oxygen and/or sulfur ring atoms present in the ring system. Alternatively, adjacent oxygen and/or sulfur ring atoms are present in the ring system. The nitrogen or sulfur atom of the heterocyclic ring system can be optionally oxidized to the corresponding N-oxide, S-oxide or S-dioxide. Non-limiting examples of suitable heterocyclic ring system include furanyl, imidazolyl, isoxazolyl, oxadiazolyl, oxazolyl, pyrazolyl, pyrrolyl, pyridyl, pyrimidyl, pyridazinyl, thiazolyl, triazolyl, tetrazolyl, thienyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, isoindolyl, acridinyl, or benzoisoxazolyl. Non-limiting examples of suitable heterocyclic rings include also aziridinyl, piperidinyl, pyrrolidinyl, piperazinyl, tetrahydropyranyl, tetrahydrofuranyl, tetrahydrothiophenyl, morpholinyl, thiomorpholinyl and the like. Also included within the scope of the term "heterocyclic" as it is used herein is a group in which the heterocyclic ring is fused at two points or joined at one point via a bond or a heteroatom linker (O, S, NH, or N(C₁-C₆alkyl), to one aromatic, or cycloalkyl ring, non-limiting examples include isoindoline-1,3-dione 1-methyl-2-phenyl-1H-pyrazole-3(2H)-one, indoline, pyridoindole and the like.

The "organic molecule", as used herein, may also refer to molecules of different classes such as small molecules conforming or not conforming to Lipinski's rule of five, (cyclic) peptides, proteins, nucleotides, lipids, sugars and derivatives thereof. In one embodiment, the organic molecule of the present invention can be selected from the group comprising small molecules conforming or not conforming to Lipinski's rule of five, (cyclic) peptides, proteins, nucleotides and mixtures thereof.

"Small" in the context of the term "organic molecule", as used herein, relates to compounds that consist of 2 or more, such as 3, 4, 5 or more carbon atoms, preferably up to 50 carbon atoms, more preferably up to 30 carbon atoms. In various embodiments, the small organic molecule has a molecular weight of at most 1500 daltons, preferably at most 700 daltons and more preferably at most 500 daltons. It may comprise heteroatoms, for example selected from O, N, and S. In various embodiments, it may be a cyclic molecule, for example a heterocyclic molecule comprising one or more heteroatoms, for example selected from N, O and S.

In various embodiments, the organic molecule used as a starter material, i.e. before it is subjected to a reaction as defined in step (2), may be selected from the following compounds:

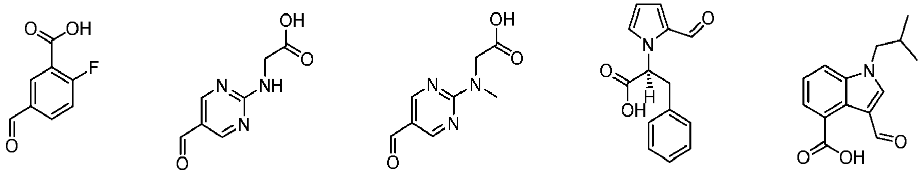

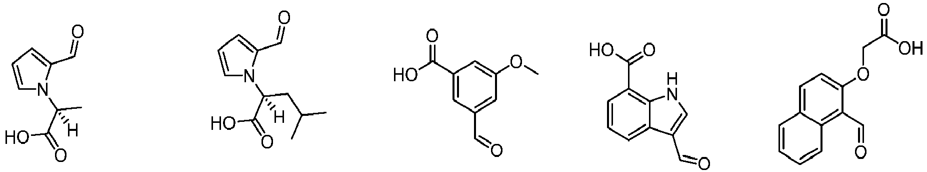

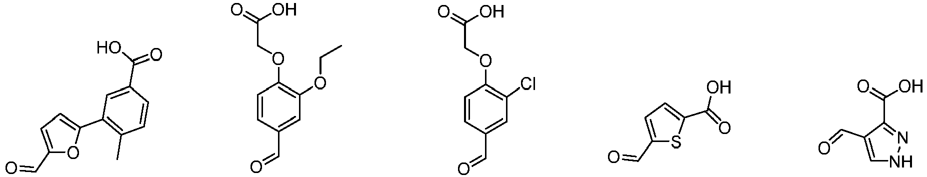

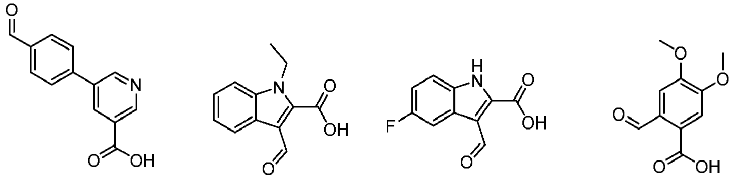

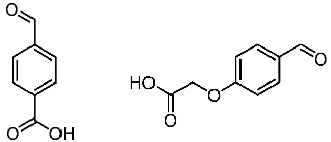

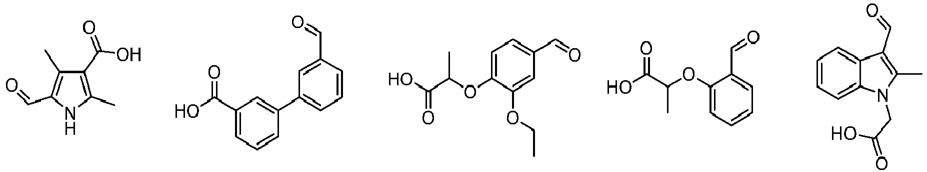

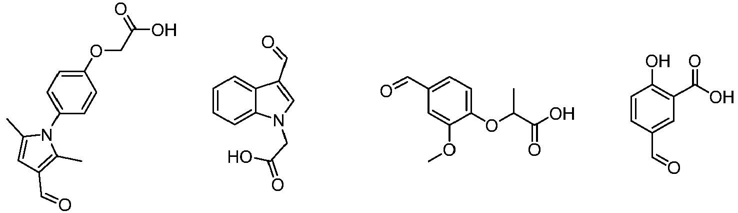

The term "nucleic acid moiety", as used herein, refers to a nucleic acid sequence that is covalently coupled to an organic moiety, as defined above, to form a conjugate molecule. The terms "nucleotide", "nucleic acid molecule" or "nucleic acid sequence", as interchangeably used herein, may relate to DNA (deoxyribonucleic acid) molecules, RNA (ribonucleic acid) molecules or molecules comprising both, DNA and RNA. The nucleic acid moiety may be single-stranded or double-stranded and may comprise secondary structures as well as regions of self-complementarity. Said molecules may appear independent of their natural genetic context and/or background. The term "nucleic acid molecule/sequence" further refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single stranded form, or a double-stranded form (helix). Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms.

"RNA" or "ribonucleic acid", as interchangeably used herein, relates to a chain of nucleotides wherein the nucleotides contain the sugar ribose and bases selected from the group of adenine (A), cytosine (C), guanine (G), or uracil (U). "DNA" or "deoxyribonucleic acid", as interchangeably used herein, relates to a chain of nucleotides wherein the nucleotides contain the sugar 2'-deoxyribose and bases selected from adenine (A), guanine (G), cytosine (C) and thymine (T). The term "mRNA" refers to messenger RNA. If DNA is used, it is preferably double-stranded, with both strands preferably having the same length and being paired by Watson-Crick base-pairing. Irrespective of whether DNA or RNA or both are used, the respective indication identifies the backbone only, as both types of nucleic acid will comprise the modified nucleotides defined herein.

The term "base" or "nucleobase", as interchangeably used herein, relates to nitrogen-containing biological compounds (nitrogenous bases) found linked to a sugar within nucleosides - the basic building blocks of deoxyribonucleic acid (DNA) and ribonucleic acid (RNA). Their ability to form base pairs and to stack upon one another lead directly to the helical structure of DNA and RNA. The primary, or canonical, nucleobases are cytosine (DNA and RNA), guanine (DNA and RNA), adenine (DNA and RNA), thymine (DNA) and uracil (RNA), abbreviated as C, G, A, T, and U, respectively. Because A, G, C, and T appear in the DNA, these molecules are called DNA-bases; A, G, C, and U are called RNA-bases. Uracil and thymine are identical except that uracil lacks the 5' methyl group. Adenine and guanine belong to the double-ringed class of molecules called purines (abbreviated as R). Cytosine, thymine, and uracil are all pyrimidines (abbreviated as Y). Other bases that do not function as normal parts of the genetic code are termed non-canonical.

The modified and/or unmodified pyrimidine nucleobases that can be used to be coupled to the organic molecule to form a compound of the library of the present invention include, but are not limited to, thymine, cytosine, uracil, 4-acetylcytidine, 5-(carboxyhydroxymethyl)uridine, 2'-O-methylcytidine, 5-carboxymethylaminomethyl-2-thiouridine, 5-carboxymethylaminomethyluridine, dihydrouridine, 2'-O-methylpseudouridine, 1-methylpseudouridine, 3-methylcytidine, 5-methylcytidine, 5-methylaminomethyluridine, 5 -methoxyaminomethyl-2-thiouridine, 5 -methoxycarbonylmethyl -2-thiouridine, 5-methoxycarbonylmethyluridine, 5-methoxyuridine, uridine-5-oxyacetic acid-methylester, uridine-5-oxyacetic acid, pseudouridine, 2-thiocytidine, 5-methyl-2-thiouridine, 2-thiouridine, 4-thiouridine, 5-methyluridine, 2'-O-alkyluridine, 2'-O-alkylthymidine, 2'-O-alkylcytidine and 3-(3-amino-3-carboxy-propyl)uridine. Also possible are combinations thereof. In some embodiments, it may be advantageous to use unmodified pyrimidine bases while in other the use of modified ones may be indicated.

The term "alkyl", as used in the context of 2'-0-alkyluridine, 2'-O-alkylthymidine, 2'-O-alkylcytidine, refers to a saturated or unsaturated hydrocarbon containing 1-20 carbon atoms including both acyclic and cyclic structures (such as methyl, ethyl, propyl, isopropyl, butyl, isobutyl, sec-butyl, pentyl, hexyl, cyclopropyl, cyclobutyl, cyclopentyl, cyclohexyl, propenyl, butenyl).

In various embodiments, the nucleic acid moiety that is coupled to the small organic molecule in the first labeling step consists of 2 to 20 nucleotides, preferably 3 to 18, 3 to 15, 3 to 12 or 3 to 10 nucleotides. The lower limit of the nucleic acid sequence at this stage may be 2, 3, 4 or 5 nucleotides. The upper limit is typically 12, 10 or 8, for example 8-12 nucleotides. The length should be selected such as to allow identification of each different conjugate/organic molecule. Said first nucleic acid sequence may be single-stranded, as for example depicted in Figures 1-4. Said nucleic acid moiety may later be elongated by addition of further nucleotides to reach its final length, as defined above. Said nucleic acid moiety used in the first labeling step may comprise only the actual barcode/code sequence and may thus consist of 7-deazapurines and/or 7-deaza-8-azapurines, and, optionally, modified and/or unmodified pyrimidine nucleotides, to avoid depurination during the following reaction steps. Further sequence elements may then be added at a later stage during the elongation steps.

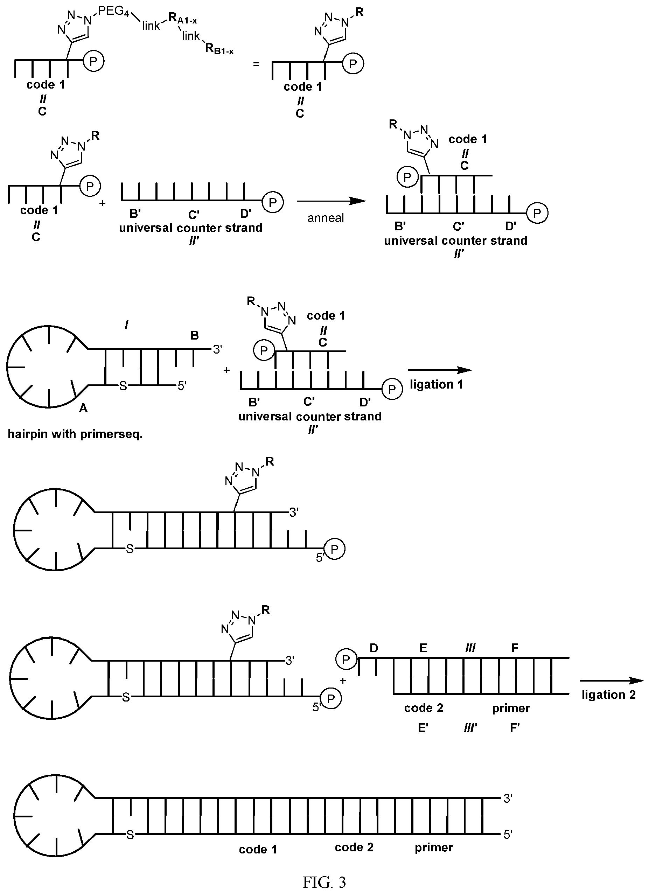

In various embodiments, the nucleic acid moiety in the final conjugate is at least 8, preferably at least 10 or more nucleotides in length. The upper limit is typically about 150 nucleotides in size, preferably up to 120, or up to 100, or up to 90, or up to 80. These size limitations do, in various embodiments, relate to the part of the nucleic acid that serves as an identifier (barcode). In addition to those, the nucleic acid moiety in the final conjugate may also comprise primer binding sites to allow amplification and/or sequencing and, optionally, other sequence elements necessary for its intended use or its generation. In various embodiments, the barcode part of the sequence may be 8 to 50 nucleotides in length, for example 8 to 40 nucleotides, while the remainder of the total length are other sequence elements not used for barcoding. The upper limits recited above are thus preferably also the upper limits for total size of the nucleic acid moiety including all sequence elements necessary for its intended use that are not the actual barcode sequence. The nucleic acid moiety in the final conjugate may be single- or double-stranded, as, for example, depicted in Figures 3-6, with double-stranded comprising embodiments where one strand forms a double-strand by self-complementarity, thus forming a hairpin structure with or without a loop.

In various embodiments, the code sequence may, for example, comprise adaptor sequences at its 5' or 3' end, preferably on both, that allow ligation of further code sequences and/or primer sequences. Said adaptor sequences may also be provided by an adaptor strand/probe that comprises a region complementary to the barcode sequence, for example also comprising inosine or abasic sites to accommodate for the modified purines, coupled to the organic moiety and additional regions, such as flanking regions that are overhanging to allow hybridization and ligation of further sequence elements. Such embodiments are exemplarily depicted in the drawings, where said complementary strand is also termed "universal counter strand". Generally, if the barcode nucleic acid moiety is single-stranded, the complementary strand may comprise inosine and/or abasic sites as well as unmodified or modified pyrimidine and, optionally, purine bases. Said complementary strand may be added after the reaction of the organic moiety is completed and elongation is desired.

In various embodiments, the nucleic acid moiety is RNA, DNA or a mixture thereof. In more preferred embodiments, the nucleic acid portion is DNA. The DNA may be double-stranded, as defined above.

In further embodiments, the present invention relates to a compound library wherein each conjugate molecule has a different organic molecule from the plurality of conjugate molecules, with each organic molecule identified by a specific nucleic acid moiety. Basically, there are two alternative methods to ensure that each molecule of the plurality of conjugate molecules comprises a nucleic acid moiety that is distinct from the nucleic acid moieties of the other conjugate molecules: first, all individual types of organic moieties are coupled to individual sequences of 7-deazapurine and/or 7-deaza-8-azapurine nucleotides and, optionally, modified/unmodified pyrimidines. By this method, the different conjugate molecules can be discriminated from each other directly after the coupling to the individual nucleic acid sequences. These sequences may include or be adaptor nucleic acids that are suitable for subsequent elongation/reaction steps that affect the nucleic acid moiety. The nucleic acid sequence may be further elongated with additional nucleotides after the synthesis steps have been applied on the organic molecule. The nucleotides used in this elongation step of the adaptor nucleic acid can include pyrimidine nucleotides and purine nucleotides, both of which can be modified and/or unmodified. The elongation step is used to individualize the nucleic acid moiety sequence attached to each individual organic molecule after it has been subjected to a reaction that led to its modification. A preferred method for the elongation of the nucleic acid moiety is DNA ligation with a ligase, such as T4 ligase, or with a chemical condensing agent, both strategies using nucleotides that comprises a nucleic acid sequence that hybridizes with the adaptor nucleic acid and that additionally comprises a nucleic acid sequence which encodes for a sequence that allows the individualization of the conjugate molecule ("second or further code sequence"). The adaptor sequences disclosed herein that may be present in the first code sequence, for example as flanking sequences, thus allow hybridization of a second nucleic acid molecule that comprises further sequence elements, such as a second or further code sequence or element necessary for functionality, and subsequently ligation in the elongation step. The to-be-ligated sequence may be a partly single-stranded hairpin structure, with the single-stranded part being used for hybridization to the nucleic acid moiety already present on the conjugate. The elongation step is thus in various embodiments a ligation step that uses a ligase to attach a further nucleotide sequence to the nucleic acid moiety of the conjugate. It has been surprisingly found that ligases, such as T4 ligase, can carry out the ligation reaction also for the modified sequences used herein for barcoding.

"Covalent" or "covalent bond", as used herein, refers to a chemical bond that involves the sharing of electron pairs between atoms. These electron pairs are known as shared pairs or bonding pairs and the stable balance of attractive and repulsive forces between atoms when they share electrons is known as covalent bonding. For many molecules, the sharing of electrons allows each atom to attain the equivalent of a full outer shell, corresponding to a stable electronic configuration. Covalent bonding includes many kinds of interactions, including σ-bonding, π-bonding, metal-to-metal bonding, agnostic interactions, bent bonds, and three-center two-electron bonds.

In various embodiments, the conjugate molecules of the library further comprise a linker portion between the nucleic acid moiety and the organic moiety molecule. In various embodiments, this linker group may be a polyoxyalkylene moiety or an alkylene moiety, in some embodiments this linker portion is polyethylene glycol (PEG). Further linker groups are described below. Chemical linkers may also be incorporated into the assembly reactants. The chemical linkers may be included between any of the moieties. Chemical linkers may optionally connect two or more of the moieties to introduce additional functionality or facilitate synthesis. The chemical linker can be a bond between any of the moieties. The bond can include a physical interaction, such as chemical bonds (either directly linked or through intermediate structures), or a non-physical interaction or attractive force, such as electrostatic attraction, hydrogen bonding, and van der Waals/dispersion forces. The linker may be conjugated to any given nucleotide within the nucleic acid moiety. In addition, any position of the ribose or the nucleobase within a given nucleotide of the nucleic acid moiety may be used to the coupling of the linker. In preferred embodiments, the linker is coupled to the nucleic acid moiety by the free 5 '-position, namely a phosphate group, of the most 5'-terminal nucleotide of the nucleic acid moiety.

The chemical linkers may aid in facilitating spatial separation of the moieties, increasing flexibility of the moieties relative to each other, introducing a cleavage site or modification site to the templated assembly reactant, facilitating synthesis of the templated assembly reactant, improving physical or functional characteristics (such as solubility, hydrophobicity, charge, cell-permeability, toxicity, biodistribution, or stability) of a templated assembly reactant, or any combination of the above. In various embodiments, the chemical linker is derived from a cross-linker that facilitates connecting the assembly reactant moieties via bioconjugation chemistry. "Bioconjugation chemistry", as used herein, refers to the chemical synthesis strategies and reagents that ligate common functional groups together under mild conditions, facilitating the modular construction of multi-moiety compounds. Due to the mild reaction conditions, bioconjugate chemistry approaches can be suitable for ligating biomolecules, such as nucleic acids, peptides, or polysaccharides. Some non-limiting examples can include chains of one or more of the following: alkyl groups, alkenyl groups, amides, esters, thioesters, ketones, ethers, thioethers, disulfides, ethylene glycol, cycloalkyl groups, benzyl groups, heterocyclic groups, maleimidyl groups, hydrazones, urethanes, azoles, imines, haloalkyl groups, carbamates, or combinations of any of these. In some embodiments, the linker is polyethylene glycol (PEG).

In addition to chemical linkers between moieties, additional functionality may optionally be introduced to the conjugate molecule by the addition of accessory groups to the moieties. Some non-limiting examples of accessory groups can include appending a chemical tag or fluorophore to track the location of the conjugation molecule, or appending an agent that improves delivery of the conjugate molecule to a given target, stabilizing groups or groups that improve purification of the conjugate molecule.

"At least one", as used herein, relates to one or more, in particular 1, 2, 3, 4, 5, 6, 7, 8, 9, 10 or more.

In a further aspect, the present invention relates to a method for synthesizing a compound library, wherein said library comprises a plurality of conjugate molecules, wherein said conjugates comprise a small organic molecule covalently coupled to a nucleic acid moiety, the synthesis of each conjugate molecule comprises: reacting a nucleic acid consisting of 7-deazapurines and/or 7-deaza-8-azapurines, and, optionally, modified and/or unmodified pyrimidine nucleotides with an organic molecule under conditions that allow the conjugation of said molecules, wherein each conjugate molecule has a different organic molecule and a different nucleic acid label that identifies its corresponding organic molecule.

As described above, the method for synthesizing a compound library may comprise the following steps: (1) reacting a nucleic acid consisting of 7-deazapurines and/or 7-deaza-8-azapurines and, optionally, modified and/or unmodified pyrimidine nucleotides with an organic molecule under conditions that allow the conjugation of said molecules; (2) subjecting the conjugate obtained in step (1) to further reactions to modify the small organic molecule; and (3) elongating the nucleic acid moiety of the conjugate obtained in step (2) by a further nucleic acid sequence. In step (1) of said method, each organic molecule is conjugated to a unique nucleic acid molecule that allows its identification. To avoid that the nucleic acid moiety is affected by the reaction in step (2), the nucleic acid moiety in step (1) consists of 7-deazapurines and/or 7-deaza-8-azapurines, and, optionally, modified and/or unmodified pyrimidine nucleotides, i.e. does not contain unmodified purine bases. The nucleic acid sequence added in step (3) can comprise modified and/or unmodified purine and pyrimidine nucleotides, as the reaction in step (2) is already completed. In various embodiments, it is also possible to repeat steps (2) and (3) multiple times. This allows synthesis of more complex organic molecules. Depending on the type of subsequent reaction the conjugate is subjected to after the first step (3), this requires that the elongation in step (3) is carried out using a suitable nucleic acid sequence, e.g. a nucleic acid sequence that consists of 7-deazapurines and/or 7-deaza-8-azapurines, and, optionally, modified and/or unmodified pyrimidine nucleotides, and only after the final reaction step (2) is completed, the elongation may be carried out with unmodified purine and, optionally pyrimidine bases.

The term "synthesizing" or "synthesis", as used herein, refers to a purposeful execution of chemical reactions to obtain a product, or several products. This happens by physical and chemical manipulations usually involving one or more reactions. This may imply that the process is reproducible, reliable, and established to work in multiple laboratories.

A chemical synthesis begins by selection of compounds that are known as reagents or reactants. Various reaction types can be applied to these to synthesize the product, or an intermediate product. This requires mixing the compounds in a reaction vessel, such as but not limited to 96-well plate, an Eppendorf tube, etc., a chemical reactor or a simple round-bottom flask. Many reactions require some form of work-up procedure before the final product is isolated. The amount of product in a chemical synthesis is the reaction yield. Typically, chemical yields are expressed as a weight in grams (in a laboratory setting) or as a percentage of the total theoretical quantity of product that could be produced. A side reaction is an unwanted chemical reaction taking place that diminishes the yield of the desired product.

"Reacting" as used with regard to the method for synthesizing a compound library refers to contacting the educts under conditions that allow formation of the product, namely the conjugate molecule. Exemplary reaction conditions are described in the examples.

In various embodiments of the method, the conjugate molecule obtained in any of the steps described above is further used in a reaction comprising a) a strong acid, preferably an acid having a pKₐ of less than 3; and/or b) a catalyst selected from the group consisting of copper, silver, gold, ruthenium, iron, osmium, cobalt, rhodium, iridium, nickel, palladium, platinum, ytterbium, and derivatives thereof, preferably copper, silver, gold, ruthenium and derivatives thereof. Said reactions may be those referred to as step (2) above.

In various embodiments of the method, the conjugate molecule is used in a reaction involving a Povarov reaction, preferably that leads to densely substituted tetrahydroquinolines; an aza-Diels-Alder reaction; a Petasis reaction; a Ugi-aza-Wittig reaction, preferably leading to oxadiazoles; a Grobke-Blackburn-Bienayme reaction; a SnAP chemistry reaction; an aminoindolizine synthesis by Au(III) catalysis; an In(III)-mediated reaction, preferably that uses carbohydrates for heterocycle synthesis.

The strength of an acid refers to its ability or tendency to lose a proton (H⁺). A strong acid is one that completely ionizes (dissociates) in a solution. In water, one mole of a strong acid HA dissolves yielding one mole of H⁺ (as hydronium ion H₃O⁺) and one mole of the conjugate base, A⁻. Essentially, none of the non-ionized acid HA remains. Examples of strong acids are hydrochloric acid (HCl), hydroiodic acid (H1), hydrobromic acid (HBr), perchloric acid (HClO₄), nitric acid (HNO₃), trifluoroacetic acid, trichloroacetic acid, and sulfuric acid (H₂SO₄). In aqueous solution, each of these essentially ionizes 100%. In contrast, a weak acid only partially dissociates. Examples in water include carbonic acid (H₂CO₃) and acetic acid (CH₃COOH). At equilibrium, both the acid and the conjugate base are present in solution. Stronger acids have a larger acid dissociation constant (Kₐ) and a smaller logarithmic constant (pKₐ = -log Kₐ) than weaker acids. Strong acids typically have a pKₐ <1.74.

The term "transition metal", as used herein, is a synonym for elements of the groups 3 to 12 of modern IUPAC numbering. Examples of transition metals are copper (Cu), silver (Ag), and gold (Au).

"Catalyst", as used herein, refers to any substance that increases the rate of a reaction without itself being consumed. In general, catalytic action is a chemical reaction between the catalyst and a reactant, forming chemical intermediates that are able to react more readily with each other or with another reactant, to form the desired end product. During the reaction between the chemical intermediates and the reactants, the catalyst is regenerated. The modes of reactions between the catalysts and the reactants vary widely. Typical of these reactions are acid-base reactions, oxidation-reduction reactions, formation of coordination complexes, and formation of free radicals.

In various embodiments, the afore-mentioned reaction conditions require that the nucleic acid moiety of the conjugate consists of 7-deazapurines and/or 7-deaza-8-azapurines and, optionally, modified and/or unmodified pyrimidine nucleotides, since unmodified purine bases may be adversely affected by said reaction conditions. Therefore, any elongation that took place before the conjugate is subjected to reaction conditions to modify the organic moiety is carried out using the appropriate nucleotides or nucleic acid sequences. Only after the organic moiety has undergone its final reaction, the last elongation step can also be carried out with unmodified purine nucleotides and, optionally, pyrimidine nucleotides.

In various embodiments of the method, the nucleic acid moiety is therefore elongated in an additional reaction step with pyrimidine and/or purine nucleotides after the conjugation step. As described above, the elongation of the nucleic acid molecule may be put into practice by using PCR and a primer nucleic acid molecule, which comprises a nucleic acid sequence that hybridizes with the adaptor nucleic acid and a nucleic acid sequence which comprises or encodes for a sequence that allows the individualization of the conjugate molecule.

It was surprisingly found that 7-deazapurines and/or 7-deaza-8-azapurines are not degraded under conditions used for the synthesis of heterocyclic chemical compounds (for example reactions involving the use of transition metals as catalysts or strong acids). However, the variety of chemical reactions carried out on the organic molecule is not limited to reactions that are only tolerated by 7-deazapurines and/or 7-deaza-8-azapurines nucleotides. This variety of chemical reactions may also comprise, but is not limited to, Povarov reaction leading to densely substituted tetrahydroquinolines, aza-Diels-Alder reaction, Petasis reaction, Ugi-aza-Wittig reaction leading to oxadiazoles, Grobke-Blackburn-Bienayme reaction, Tin Amine Protocol (SnAP) chemistry, Aminoindolizine synthesis by Au(III) catalysis, an In(III)-mediated reaction that uses carbohydrates for heterocycle synthesis, amide synthesis, stepwise substitution of cyanuric chloride, protective group chemistry, amide bond formation, carbonyl reactions, such as synthesis of diamine-containing cyclic or linear compounds, aromatic substitutions, such as nucleophilic aromatic substitutions of reactive heteroaromatic halides, cross-coupling reactions, such as palladium-catalyzed synthesis of biaryl compounds, Diels-Alder reaction, benzimidazole formation, macrocycle synthesis, substitution with N-nucleophiles, nucleophilic substitution of reactive aliphatic halides, Cu(I)-catalyzed alkyne-azide cycloaddition, Horner-Wadsworth-Emmons reation with reactive phosphonium ylids, reduction of aromatic nitro groups and appendage of cyanuric chloride.

In a third aspect, the present invention relates to the use of a compound library of the present invention for screening a compound binding to a target molecule.

The term "screening", as used herein, means that a plurality of candidate compounds (this can be an amount of 10 or less compounds or up to 10.000.000 compounds or more) can be screened in a single experiment for their ability to bind to a given target molecule. In various embodiments, 10 or more, 20 or more, 50 or more, 100 or more, 1000 or more, 1500 or more, 2000 or more, 5000 or more or 10000 or more of the candidate compounds are pooled in a single vessel and tested for their ability to bind to target molecule. Thus, said candidate compounds pooled in a single vessel and exposed to the target molecule compete for binding to said target molecule. Upon being brought into contact with the target molecule, the candidate compounds may bind to the target molecule or being washed out by one or more washing steps. Subsequently, the candidate compounds bound to the target molecule can be identified via their individual DNA label. Methods to identify candidate compounds based on their DNA label are described herein and are well-known in the art.

A "target molecule", as used herein, may be any molecule which can be bound by a binding molecule and may for example be a biological substance such as a biomolecule, complexes, cell fractions or cells. Preferably, a target molecule within the context of the present invention is a protein and/or a nucleic acid, e.g. DNA, or RNA molecule. Even more preferred are target molecules such as a peptide, a protein, a drug molecule, a small molecule, or a vitamin. In some preferred embodiments of the present invention, a "target molecule" may be a biomarker. A target molecule may also refer to a biological molecule that is either naturally present in a cell or subject or has been previously introduced into a cell or subject. A "target molecule" can be any polypeptide, lipid, nucleic acid, small molecule, saccharide or a vitamin in, on or of the cell. It may be located in the nucleus, nucleolus, cytoplasm, mitochondria, Golgi apparatus, endoplasmic reticulum or membrane of the cell. In various embodiments, the target molecules of the methods of the present invention are polypeptide molecules. In various embodiments, the target molecule is a protein.

The term "protein", as used herein, relates to one or more associated polypeptides, wherein the polypeptides consist of amino acids linked by peptide (amide) bonds. The term polypeptide refers to a polymeric compound comprised of covalently linked amino acid residues. The amino acids are preferably the 20 naturally occurring amino acids glycine, alanine, valine, leucine, isoleucine, phenylalanine, cysteine, methionine, proline, serine, threonine, glutamine, asparagine, aspartic acid, glutamic acid, histidine, lysine, arginine, tyrosine and tryptophan.

Suitable targets may include one or more of peptides, proteins (e.g., antibodies, affibodies, or aptamers), nucleic acids (e.g., polynucleotides, DNA, RNA, or aptamers); polysaccharides (e.g., lectins or sugars), lipids, enzymes, enzyme substrates, ligands, receptors, antigens, or haptens. One or more of the aforementioned targets may be characteristic for particular cells, while other targets may be associated with a particular disease or condition. In some embodiments, targets in a tissue sample that may be detected and analyzed using the methods disclosed herein may include, but are not limited to, prognostic targets, hormone or hormone receptor targets, lymphoid targets, tumor targets, hematopoietic targets, cell cycle associated targets, neural tissue and tumor targets, or cluster differentiation targets.

Suitable examples of prognostic targets may include enzymatic targets such as galactosyl transferase II, neuron specific enolase, proton ATPase-2, or acid phosphatase.

Suitable examples of hormone or hormone receptor targets may include human chorionic gonadotropin (HCG), adrenocorticotropic hormone, carcinoembryonic antigen (CEA), prostate-specific antigen (PSA), estrogen receptor, progesterone receptor, androgen receptor, gClq-R/p33 complement receptor, IL-2 receptor, p75 neurotrophin receptor, PTH receptor, thyroid hormone receptor, or insulin receptor.

Suitable examples of hematopoietic targets may include CD45, CD34, CD133, HLA-DR, CD115, CD116, CD117, CD33, CD38, CD90, CD71, Ki67, Flt3, CD163, CD45RA, CD3, IgD, CD105, CD45, and also c-kit, - the receptor for stem cell factor.

Suitable examples of lymphoid targets may include alpha-1-antichymotrypsin, alpha-1-antitrypsin, B cell target, bcl-2, bcl-6, B lymphocyte antigen 36 kD, BM1 (myeloid target), BM2 (myeloid target), galectin-3, granzyme B, HLA class I Antigen, HLA class II (DP) antigen, HLA class II (DQ) antigen, HLA class II (DR) antigen, human neutrophil defensins, immunoglobulin A, immunoglobulin D, immunoglobulin G, immunoglobulin M, kappa light chain, kappa light chain, lambda light chain, lymphocyte/histocyte antigen, macrophage target, muramidase (lysozyme), p80 anaplastic lymphoma kinase, plasma cell target, secretory leukocyte protease inhibitor, T cell antigen receptor (JOVI 1), T cell antigen receptor (JOVI 3), terminal deoxynucleotidyl transferase, or unclustered B cell target.

Suitable examples of tumor targets may include alpha fetoprotein, apolipoprotein D, BAG-I (RAP46 protein), CA19-9 (sialyl lewisa), CA50 (carcinoma associated mucin antigen), CA125 (ovarian cancer antigen), CA242 (tumor associated mucin antigen), chromogranin A, clusterin (apolipoprotein J), epithelial membrane antigen, epithelial-related antigen, epithelial specific antigen, gross cystic disease fluid protein- 15, hepatocyte specific antigen, heregulin, human gastric mucin, human milk fat globule, MAGE-I, matrix metalloproteinases, melan A, melanoma target (HMB45), mesothelin, metallothionein, microphthalmia transcription factor (MITF), Muc-1 core glycoprotein. Muc-1 glycoprotein, Muc-2 glycoprotein, Muc-5AC glycoprotein, Muc-6 glycoprotein, myeloperoxidase, Myf-3 (Rhabdomyosarcoma target), Myf-4 (Rhabdomyosarcoma target), MyoDl (Rhabdomyosarcoma target), myoglobin, nm23 protein, placental alkaline phosphatase, prealbumin, prostate specific antigen, prostatic acid phosphatase, prostatic inhibin peptide, PTEN, renal cell carcinoma target, small intestinal mucinous antigen, tetranectin, thyroid transcription factor- 1, tissue inhibitor of matrix metalloproteinase 1, tissue inhibitor of matrix metalloproteinase 2, tyrosinase, tyrosinase-related protein-1, villin, or von Willebrand factor.

Suitable examples of cell cycle associated targets may include apoptosis protease activating factor- 1, bcl-w , bcl-x, bromodeoxyuridine, CAK (cdk-activating kinase), cellular apoptosis susceptibility protein (CAS), caspase 2, caspase 8, CPP32 (caspase-3), CPP32 (caspase-3), cyclin dependent kinases, cyclin A, cyclin Bl, cyclin D1, cyclin D2, cyclin D3, cyclin E, cyclin G, DNA fragmentation factor (N-terminus), Fas (CD95), Fas-associated death domain protein, Fas ligand, Fen-1, IPO-38, McI-I, minichromosome maintenance proteins, mismatch repair protein (MSH2), poly (ADP- Ribose) polymerase, proliferating cell nuclear antigen, pl6 protein, p27 protein, p34cdc2, p57 protein (Kip2), pi 05 protein, Stat 1 alpha, topoisomerase I, topoisomerase II alpha, topoisomerase III alpha, or topoisomerase II beta.

Suitable examples of neural tissue and tumor targets may include alpha B crystallin, alpha-internexin, alpha synuclein, amyloid precursor protein, beta amyloid, calbindin, choline acetyltransferase, excitatory amino acid transporter 1, GAP43, glial fibrillary acidic protein, glutamate receptor 2, myelin basic protein, nerve growth factor receptor (gp75), neuroblastoma target, neurofilament 68 kD, neurofilament 160 kD, neurofilament 200 kD, neuron specific enolase, nicotinic acetylcholine receptor alpha4, nicotinic acetylcholine receptor beta2, peripherin, protein gene product 9, S- 100 protein, serotonin, SNAP-25, synapsin I, synaptophysin, tau, tryptophan hydroxylase, tyrosine hydroxylase, or ubiquitin.

Suitable examples of cluster differentiation targets may include CDIa, CDIb, CDIc, CDId, CDIe, CD2, CD3delta, CD3epsilon, CD3gamma, CD4, CD5, CD6, CD7, CD8alpha, CD8beta, CD9, CD10, CD1 la, CD1 Ib, CD1 Ic, CDw12, CD13, CD14, CD15, CD15s, CD16a, CD16b, CDwl7, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42a, CD42b, CD42c, CD42d, CD43, CD44, CD44R, CD45, CD46, CD47, CD48, CD49a, CD49b, CD49c, CD49d, CD49e, CD49f, CD50, CD51, CD52, CD53, CD54, CD55, CD56, CD57, CD58, CD59, CDw60, CD61, CD62E, CD62L, CD62P, CD63, CD64, CD65, CD65s, CD66a, CD66b, CD66c, CD66d, CD66e, CD66f, CD68, CD69, CD70, CD71, CD72, CD73, CD74, CDw75, CDw76, CD77, CD79a, CD79b, CD80, CD81, CD82, CD83, CD84, CD85, CD86, CD87, CD88, CD89, CD90, CD91, CDw92, CDw93, CD94, CD95, CD96, CD97, CD98, CD99, CD100, CD101, CD102, CD103, CD104, CD105, CD106, CD107a, CD107b, CDw108, CD109, CD1 14, CD1 15, CD1 16, CD1 17, CDwI 19, CD120a, CD120b, CD121a, CDw121b, CD122, CD123, CD124, CDw125, CD126, CD127, CDw128a, CDw128b, CD130, CDw131, CD132, CD134, CD135, CDw136, CDw137, CD138, CD139, CD140a, CD140b, CD141, CD142, CD143, CD144, CDw145, CD146, CD147, CD148, CDw149, CDw150, CD151, CD152, CD153, CD154, CD155, CD156, CD157, CD158a, CD158b, CD161, CD162, CD163, CD 164, CD 165, CD 166, and TCR-zeta.

Other suitable prognostic targets hormone or hormone receptor targets lymphoid targets tumor targets cell cycle associated targets neural tissue and tumor targets include centromere protein-F (CENP-F), giantin, involucrin, lamin A&C (XB 10), LAP-70, mucin, nuclear pore complex proteins, pi 80 lamellar body protein, ran, cathepsin D, Ps2 protein.

The term "contacting", as used herein in the context of conjugate molecules and target molecules, refers generally to providing access of one component, reagent, analyte or sample to another. For example, contacting can involve mixing a solution comprising a conjugate molecule and a target molecule. The solution comprising one component, reagent, analyte or sample may also comprise another component or reagent, such as dimethyl sulfoxide (DMSO) or a detergent, which facilitates mixing, interaction, uptake, or other physical or chemical phenomenon advantageous to the contact between components, reagents, analytes and/or samples.

The term "identify", as used herein in the context of screening, relates to the recognition of a conjugate molecule that has the ability to completely or partially bind to the biological target molecule, and/or completely or partly revert molecular changes of a cell induced by pathological conditions or to inhibit or activate enzymatic functions.

The term "sequence", as used herein, relates to the primary nucleotide sequence of nucleic acid molecules or the primary amino acid sequence of a protein.

**FIG. 1** shows the synthesis of target molecules on controlled pore glass-coupled DNA barcodes (two or three-cycle library). The designation of the sequence code 1 may be found in Tables 2-4, and code 1 identifies building block R_{A1-x}; FG is Functional group, and CPG is Controlled pore glass; the cleavage occurs with aqueous ammonia or aqueous methylamine.

The CPG-bound oligonucleotide has a linker (e.g. PEG depicted here) between the nucleic acid moiety and a functional group 'FG'. A building block R_{A1-X} coupled to FG₁ and is then added to the CPG-bound oligonucleotide where FG₁ replaces the FG, and FG₁ functions as a 'link' between the PEG and the building block R_{A1-x}, A second building block is coupled similarly to the first building block where the FG1 of the second building block functions as a 'link' between the first building block and the second building block. Two building blocks are depicted here (known as a 2-cycle library), but a 3-cycle library is also possible. Up to 5 building blocks, i.e. a 5-cycle library may also be useful with the compound library disclosed herein. The controlled pore glass is then cleaved after the addition of building blocks is complete.

**FIG. 2** shows the enzymatic phosphorylation of chemically modified code 1 DNA sequences ***II*** and degenerate counter strand *II*' using a polynucleotide kinase (PNK). Once the building blocks have been added, it is phosphorylated at the 5'-end with a polynucleotide kinase, such as T4 PNK in a non-limiting example. A universal counter strand II' is depicted that is also phosphorylated at its 5'-end.

The universal counter strand II' is then annealed to the oligonucleotide sequence with the building blocks as depicted in FIG. 3. **FIG. 3** illustrates a two-cycle encoding strategy for short single-stranded DNA conjugates. The designation of the sequences may be found in Tables 2-4; R is PEG(4)-amine, and S is triethylene glycol spacer. A primer sequence is then added (here depicted as a hairpin primer sequence) to elongate the annealed sequence. A second elongation (e.g. ligation) occurs to add a code 2 with a primer sequence, but such elongation may occur as many times as desired by one skilled in the art. The elongation may occur with additional nucleotides that may be pyrimidines and/or purines, both of which may be modified and/or unmodified. For example, the pyrimidines and/or purines of the hairpin with primer sequence and/or primer of code 2 may be modified and/or unmodified in an embodiment. The elongation of the sequence allows for individualization of the nucleic acid moiety sequence attached to each individual organic molecule. In a non-limiting embodiment, T4 ligase and/or a chemical condensing agent may be used for elongation purposes.

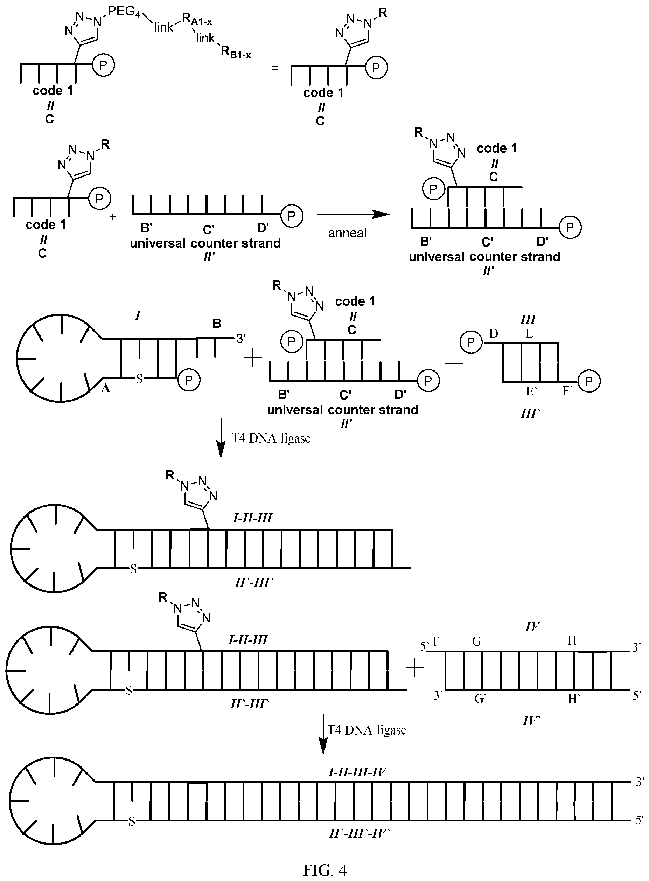

**FIG. 4** shows a hairpin (three cycle) encoding strategy for short single-stranded DNA conjugates. The designation of the sequences may be found in Tables 3-4. The process depicted in FIG. 4 is similar to that of FIG. 3 but two additional sequences are ligated to the annealed strand comprising the phosphorylated code 1 strand and the phosphorylated universal counter strand II'.

More specific examples of the above procedures are noted below, with respect to the Examples.

### EXAMPLES

### Example 1a: Synthesis of the 7-deazaadenine building block for automated DNA synthesis

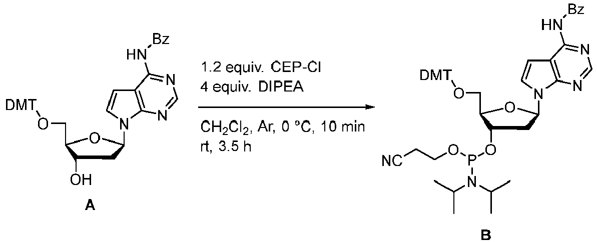

N⁶-Benzoyl-2'-deoxy-5'-O-DMT-7-deaza-2'-deoxyadenosine 3'-CE phosphoramidite was synthesized. Before setting up the reaction, N⁶-Benzoyl-2'-deoxy-5'-O-DMT-7-deaza-2'-deoxyadenosine was dried in high vacuum (1 mbar, 0.1 kPa) overnight. CEP-C1 (215 µL, 0.91 mmol, 1.2 equiv.) under Argon at 0 °C was added to the stirred solution of N⁶-Benzoyl-2'-deoxy-5'-O-DMT-7-deaza-2'-deoxyadenosine (500 mg, 0.76 mmol) in dry dichloromethane (5 mL) and DIPEA (523 µL, 3.05 mmol, 4 equiv.). The cooling bath was removed after 10 minutes and the solution was stirred at ambient temperature for 3.5 hours. The solution was filtered through a syringe filter and diluted with 5 mL dichloromethane. The organic phase was washed with a saturated aqueous solution of NaHCO3 (2x 10 mL) and brine (10 mL), then dried over anhydrous MgSO₄, filtered and concentrated *in vacuo.*

### Example 1b: Synthesis of N6-DMF-2'-deoxy-5'-O-DMT-7-deaza-8-aza-2'-deoxyadenosine

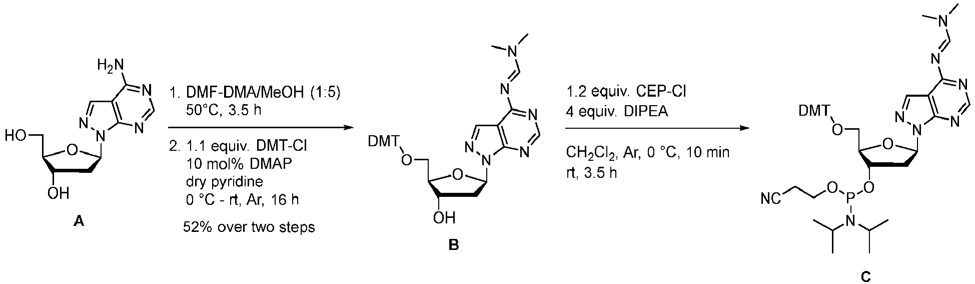

7-deaza-8-aza-2'-deoxyadenosine was dried in high vacuum (1 mbar, 0.1 kPa) overnight before setting up the reaction. Step 1: The solution of 7-deaza-8-aza-2'-deoxyadenosine (500 mg, 2.00 mmol) in dry methanol (10 mL) and DMF-DMA (2 mL) was stirred at 50°C for 3.5 hour. The solvents were removed under at 10 mbar (1 kPa). The orange residue was coevaporated twice with each 5 mL dry methanol and 5 mL diethyl ether, dried under vacuum, and then immediately used in Step 2.

Step 2: DMAP (22.6 mg, 0.20 mmol, 10 mol%, in 0.4 mL dry pyridine) and DMT-C1 (752.1 mg, 2.20 mmol, 1.1 equiv., in 3.6 mL dry pyridine) at 0°C under argon were added to the solution of *N*₆-DMF-2'-deoxy-7-deaza-8-azaadenosine (612 mg, 2.00 mmol) in dry pyridine (6 mL). The solution was stirred overnight allowing it to warm up to 25°C. The solvent was removed under reduced pressure and the residue was dissolved in 100 mL dichloromethane. The organic layer was washed with ice-cold brine (0°C, 3 x 50 mL) and ice-cold water (0°C, 1 x 50 mL), dried over MgSO₄ and removed under reduced pressure. The product was purified by silica gel column chromatography using methanol/ dichloromethane as eluent.

### Example 1c: Synthesis of N⁶-DMF-2'-deoxy-5'-O-DMT-2'-7-deaza-8-aza-2'-deoxyadenosine 3'-CE phosphoramidite

N⁶-DMF-2'-deoxy-5'-O-DMT-2'-7-deaza-8-aza-2'-deoxyadenosine was dried in high vacuum overnight before setting up the reaction. CEP-C1 (278 µL, 1.23 mmol, 1.2 equiv.) under Argon at 0 °C was added to the stirred solution of N⁶-DMF-2'-deoxy-5'-O-DMT-2'-7-deaza-8-aza-2'-deoxyadenosine (625 mg, 1.03 mmol) in dry dichloromethane (9 mL) and DIPEA (706 µL, 4.11 mmol, 4 equiv.). The cooling bath was removed after 10 minutes and the solution was stirred at ambient temperature for 3.5 hours. The solution was filtered through a stringe filter and diluted with 15 mL dichloromethane. The organic phase was washed with a saturated aqueous solution of NaHCO3 (2x 30 mL), brine (30 mL) then dried over anhydrous MgSO₄, filtered and concentrated *in vacuo.*

### Synthesis of the 7-deaza-8-aza-guanine building block for automated DNA synthesis

### Example 2: Synthesis of N6-DMF-2'-deoxy-5'-O-DMT-7-deaza-8-aza-2'-deoxyguanosine.

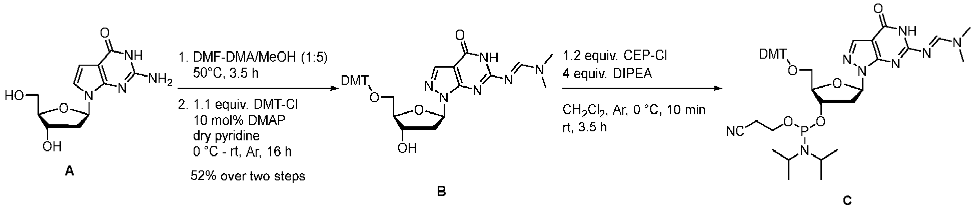

7-deaza-8-aza-2'-deoxyguanosine was dried in high vacuum overnight was dried before setting up the reaction.
Step 1: The solution of 7-deaza-8-aza-2'-deoxyguanosine (500 mg, 2.00 mmol) in dry methanol (10 mL) and DMF-DMA (2 mL) was stirred at 50°C for 3.5 hours. The solvents were removed under at 10 mbar (1 kPa). The orange residue was coevaporated twice with each 5 mL dry methanol and 5 mL diethyl ether, dried under vacuum, and then immediately used in the next step.
Step 2: DMAP (22.6 mg, 0.20 mmol, 10 mol%, in 0.4 mL dry pyridine) and DMT-C1 (752.1 mg, 2.20 mmol, 1.1 equiv., in 3.6 mL dry pyridine) at 0°C under argon were added to the solution of *N*₆-DMF-2'-deoxy-7-deaza-8-azaguanosine (612 mg, 2.00 mmol) in dry pyridine (6 mL). The solution was stirred overnight allowing it to warm up to 25°C. The solvent was removed under at 10 mbar (1 kPa), and the residue was dissolved in 100 mL dichloromethane. The organic layer was washed with ice-cold brine (0°C, 3 x 50 mL) and ice-cold water (0°C, 1 x 50 mL), dried over MgSO₄ and removed under reduced pressure. The product was purified by silica gel column chromatography using methanol/ dichloromethane as eluent.

### Example 2b: Synthesis of N⁶-DMF-2'-deoxy-5'-O-DMT-2'-7-deaza-8-aza-2'-deoxyguanosine 3'-CE phosphoramidite

N⁶-DMF-2'-deoxy-5'-O-DMT-2'-7-deaza-8-aza-2'-deoxyguanosine was dried in high vacuum overnight before setting up the reaction. CEP-C1 (278 µL, 1.23 mmol, 1.2 equiv.) under Argon at 0 °C was added to the stirred solution of N⁶-DMF-2'-deoxy-5'-O-DMT-2'-7-deaza-8-aza-2'-deoxyguanosine (625 mg, 1.03 mmol) in dry dichloromethane (9 mL) and DIPEA (706 µL, 4.11 mmol, 4 equiv.). The cooling bath was removed after 10 minutes, and the solution was stirred at ambient temperature for 3.5 hours. The solution was filtered through a stringe filter and diluted with 15 mL dichloromethane. The organic phase was washed with a saturated aqueous solution of NaHCO3 (2x 30 mL) and brine (30 mL), then dried over anhydrous MgSO₄, filtered and concentrated *in vacuo.*

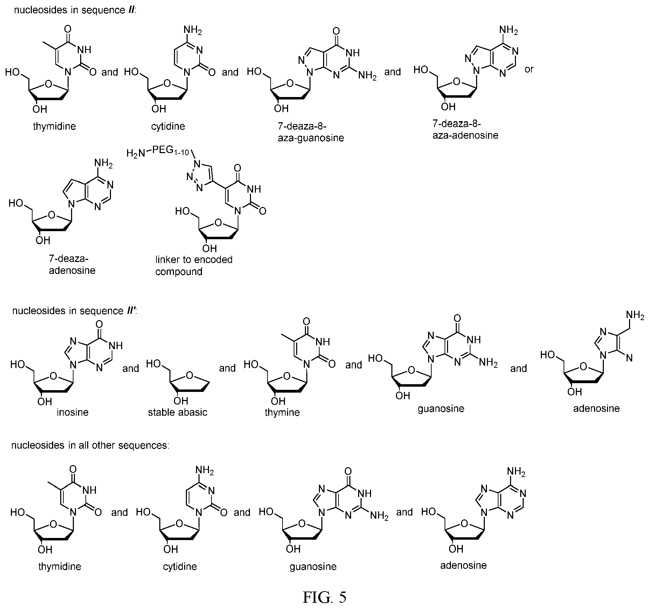

**FIG. 5** illustrates the stabilized purines usable for automated DNA oligonucleotide synthesis and screening of the DNA oligonucleotides for stability. **Examples 1a-1c and 2a-2**b illustrate the synthesis of stabilized purines from readily available precursors, which obviates lengthy nucleoside synthesis; the stabilized purines can also be converted to phosphoramidites for automated DNA oligonucleotide synthesis in three, high-yielding steps without chromatography. Furthermore, the chemically modified phosphoramidites give comparable yields in DNA synthesis.

The chemical stability of DNA sequences was evaluated as previously described, experimental details are given below.

### Example 3a: Treatment of solid support-coupled DNA with acids

20 nmol of controlled pore glass (CPG)-coupled oligonucleotide were treated with 50 µL of an acid according to Table 1 (e.g. 10 % trifluoroacetic acid). The suspension was shaken at ambient temperature for 22 h. Afterwards, the solution was removed by filtration, and the CPG was washed three times with each 200 µL of 0.1 M MgCl₂ solution, water, DMF, MeOH, CAN, and CH₂Cl₂ and dried in vacuo.

Cleavage and analysis: DNA was deprotected and cleaved from CPG by shaking the DNA in 500 µL of an AMA solution (AMA = aqueous ammonia (30%)/ aqueous methylamine (40%), 1:1, vol/vol) for 4 h at room temperature. Afterwards 20 µL of 1 M Tris buffer (pH = 7.5) were added, and the mixture was dried under at 10 mbar (1 kPa) (SpeedVac) and dissolved in 200 µL of distilled water. The product was analyzed by analytical RP-HPLC and MALDI-TOF-MS.

### Example 3b: Treatment of solid support-coupled DNA with metal salts

20 nmol of CPG-coupled oligonucleotide were treated with 200 equiv. of metal salt (4 µmol) dissolved in 50 µL dry solvent. The suspension was shaken at ambient temperature for 22 h. Afterwards, the solvent was removed by filtration, and the CPG was washed three times with each 200 µL of 0.1 M EDTA solution, 0.1 M MgCl₂ solution, water, DMF, MeOH, ACN and CH₂Cl₂ and dried in vacuo.

Cleavage and analysis: DNA was deprotected and cleaved from the CPG by shaking with 500 µL of an AMA solution (AMA = aqueous ammonia (30%)/ aqueous methylamine (40%), 1:1, vol/vol) for 4 h at room temperature. Afterwards, 20 µL of 1 M Tris buffer (pH = 7.5) were added, and the mixture was dried at 10 mbar (1 kPa) (SpeedVac), and the DNA was dissolved in 200 µL distilled water. The product was analyzed by Analytical RP-HPLC and MALDI-TOF-MS.

### Example 3c: Treatment of solid support-coupled DNA with organocatalysts

20 nmol of CPG-coupled oligonucleotide (ca. 0.7 mg of solid phase) were treated with 200 equiv. of organocatalyst (4 µmol) dissolved in 50 µL dry solvent. The suspension was shaken at ambient temperature for 22 h. Afterwards, the solvent was removed by filtration, and the CPG was washed three times with each 0.1 M MgCl₂ solution, water, DMF, MeOH, ACN and CH₂Cl₂ and dried in vacuo.

Cleavage and analysis: DNA was deprotected and cleaved from CPG by shaking in 500 µL of an AMA solution (AMA = aqueous ammonia (30%)/ aqueous methylamine (40%), 1:1, vol/vol) for 4 h at room temperature. Afterwards, 20 µL of 1 M Tris buffer (pH = 7.5) were added, the mixture was dried at 10 mbar (1 kPa) (SpeedVac), and the DNA was dissolved in 200 µL distilled water. The product was analyzed by Analytical RP-HPLC and MALDI-TOF-MS.

**Table 1 - Stability of stabilized DNA against aqueous acids, metal salts, and organic reagents.^{a}. The percentages represent the amount of DNA degradation.**

| **Entry** | **Metal salt** | **Solvent** | **ATC** | **A*TC** | **A**TC** |
|---|---|---|---|---|---|
| **1** | 10% TFA | | > 61% | 0-20% | 0-20% |
| 2 | 3.7% HCl | | > 61% | 0-20% | 0-20% |
| 3 | Bi(OTf)₃ | MeOH | 0-20% | 0-20% | 0-20% |
| 4 | Ce(NH₄)₂(NO₃)₆ | MeOH | > 61% | 0-20% | 0-20% |
| 5 | Co(acac)₃ | ACN | 0-20% | 0-20% | 0-20% |
| 6 | Cu(MeCN)₄PF₆ | ACN | 21-40% | 0-20% | 0-20% |
| 7 | FeCl₂ •4 H₂O | ACN | 0-20% | 0-20% | 0-20% |
| 8 | La(O*i*-Pr)₃ | THF | 0-20% | 0-20% | 0-20% |
| 9 | LiBr | ACN | 0-20% | 0-20% | 0-20% |
| 10 | Ni(acaC)₂ | ACN | 0-20% | 0-20% | 0-20% |
| 11 | Pd(OAc)₂ | ACN | > 61% | > 61% | > 61% |
| 12 | RuCl3 | ACN | 41-60% | 21-40% | 21-40% |
| 13 | [Ru(*p-*cymene)Cl₂]₂ | CH₂Cl₂ | 21-40% | 21-40% | 0-20% |
| 14 | Grubbs 1^{st} Gen. | CH₂Cl₂ | 21-40% | 0-20% | 0-20% |
| 15 | Sc(OTf)₃ | ACN | 0-20% | 0-20% | 0-20% |
| 16 | Sc(OTf)₃, 40 °C | ACN | 41-60% | 21-40% | 21-40% |
| 17 | SeO₂ | MeOH | 0-20% | 0-20% | 0-20% |
| 18 | Yb(OTf)₃ | MeOH | 0-20% | 0-20% | 0-20% |
| 19 | ZnCl₂ | ACN | 0-20% | 0-20% | 0-20% |
| 20 | DDQ | EtOH | 21-40% | 0-20% | 0-20% |
| 21 | PIDA | ACN | 41-60% | 0-20% | 0-20% |
| 22 | TEMPO | ACN | 0-20% | 0-20% | 0-20% |

| | | | | | |
|---|---|---|---|---|---|
| ^{a} for each: 20 nmol DNA, 200 eq. metal salt or organic reagent, 50 µL solvent, r.t., 22 h. ACN = acetonitrile, MeOH = methanol, THF = tetrahydrofuran, ATC = 5'-TTA CTA CCT A-3'-CPG (SEQ ID NO:1), A*/**TC = 5'-TT A*/** CT A*/** CCA*/** T-3'-CPG, A* = 7-deaza-2'-deoxyadenosine (SEQ ID NO:2), A** = 7-deaza-8-aza-2'-deoxyadenosine (SEQ ID NO:3). | | | | | |

A systematic screening of DNA stability in presence of aqueous acids, metal salts and organic reagents revelead that an ATC-sequence was strongly degraded in the presence of strong protic acids (see table 1, entries 1-2), several metals (table 1, entries 11-12) or metals with a high redox potential (see table 1, entry 4). Higher temperature in combination with metals also led in several cases to high degrees of DNA degradation compared to the results under ambient temperature (table 1, entries 15-16).^{Chem.Sci.2019} Chemically stabilized DNA-strands containing 7-deaza- or 7-deaza-8-azaadenosine revealed a much higher stability under these conditions than the native DNA. For example, the treatment of the chemically modified DNA-strands with strong protic acids lead to no DNA-degradation (see table 1, entries 1-2). Also in the presence of Ce(III) with a high redox potential, or the hypervalent iodine oxidant PIDA, the chemically modified DNA was completely stable (see table 1, entries 4 and 21). Thus, the usage of 7-deaza- as well as 7-deaza-8-azaadenosine increased the stability of DNA. Thus, the 8-aza-7-deaza-purines are compatible with established DNA-barcoding strategies relying on double-stranded DNA and offer a desired stability profile.

### Example 4: Exemplary Library synthesis

### Example 4a: Copper(I)-catalyzed alkyne-azide cycloaddition to install a linker moiety on chemically stabilized code I

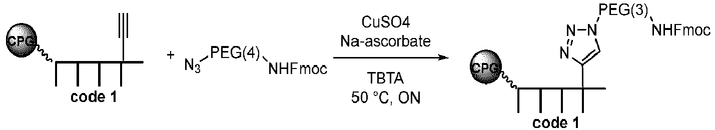

The CPG-bound oligonucleotide alkyne conjugate (50 nmol) was suspended in 100 µL DMF and diluted with 380 µL of H₂O/MeOH (1:1). Subsequently, the azide (125 µmol, 2500 equiv.) was dissolved in 100 µL of DMF/H₂O (9:1), TBTA (6.25 µmol, 125 equiv.) dissolved in 20 µL of DMF, Na-ascorbate (6.25 µmol, 125 equiv.) dissolved in 10 µL of H₂O, and CuSO₄^{∗}5H₂O (0.625 µmol, 12.5 equiv.) dissolved in 10 µL of H₂O were added to the suspension in this order. Stock solutions of all reactants were prepared before the reaction was started. The reaction mixtures were shaken at 50 °C overnight. Then the CPG-bound conjugate was filtered over a filter column and washed three times with each 200 µL of 0.1 M EDTA solution, 0.1 M MgCl₂ solution, water, DMF, MeOH, ACN and CH₂Cl₂ and dried *in vacuo.* The completeness of the reaction was controlled by cleavage of a small portion (∼20 nmol) of CPG-bound oligonucleotide conjugate with 500 µL AMA (AMA = aqueous ammonia (30 %)/ aqueous methylamine (40 %), 1:1, vol/vol) for 4 h at ambient temperature. To this solution 20 µL of 1 M Tris buffer (pH = 7.5) were added, the mixture was dried in a SpeedVac, re-dissolved in 200 µL of distilled water. The crude was analyzed by analytical RP-HPLC (Method II) and MALDI-MS.

### Example 4b: Amide coupling to couple a bifunctional starting material to the code I

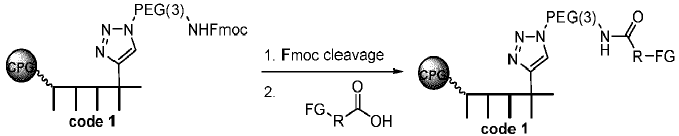

### Protocol:

**Step 1:** The Fmoc-protecting group of the CPG-bound oligonucleotide (250 nmol) was cleaved off by addition of 200 µL 20 % piperidine in dry DMF and shaken for 5 min. Afterwards, the CPG-bound deprotected oligonucleotide was washed three times with each 200 µL of DMF, MeOH, ACN and CH₂Cl₂ and then dried in vacuo.

### Step 2:

CPG-coupled oligonucleotide, carboxylic acid and HATU were dried *in vacuo* for 15 min. Stock solutions of all reactants in dry DMF were prepared before the reaction was started. HATU (25 µmol, 100 equiv.) dissolved in 75 µL dry DMF and DIPEA (62.5 µmol, 250 equiv.) were added to the solution of carboxylic acid (25 µmol, 100 equiv.) in 75 µL dry DMF. The mixture was shaken for 5 min and added to CPG-coupled DNA suspended in 75 µL dry DMF (250 nmol, 1 equiv.). The amide coupling reaction was shaken at ambient temperature for 2 hours. Next, the CPG-coupled conjugate was filtered over a filter column, washed three times with each 200 µL of DMF, MeOH, ACN and CH₂Cl₂ and dried *in vacuo.* Amide coupling was repeated two times.

Completion of amide coupling was checked by cleaving off a 5 % of CPG-coupled oligonucleotide conjugate (0.7-0.9 mg, ∼20 nmol) with 500 µL AMA (AMA = aqueous ammonia (30 %)/ aqueous methylamine (40 %), 1:1, vol/vol) for 30 min (hexT) or 4 h (ATGC-sequences) at ambient temperature. Afterwards, 20 µL of 1 M Tris buffer (pH = 7.5) were added, the mixture was dried under reduced pressure (SpeedVac) and DNA was dissolved in 200 µL distilled water. The crude reaction mixture was analyzed by analytical RP-HPLC and MALDI-MS. In the case of uncompleted coupling (<90%) the reaction was repeated a third time.

Unreacted amines were capped with acetic acid anhydride (three times 200 µL, 30 s, 1:1 mixture of THF/methylimidazole, 9:1, vol/vol, and THF/pyridine/acetic acid anhydride 8:1:1, vol/vol). The capped CPG-coupled oligonucleotide conjugate was washed three times with each 200 µL of DMF, MeOH, ACN and CH₂Cl₂ and dried *in vacuo.*

### EXAMPLES OF BIFUNCTIONAL STARTING MATERIALS

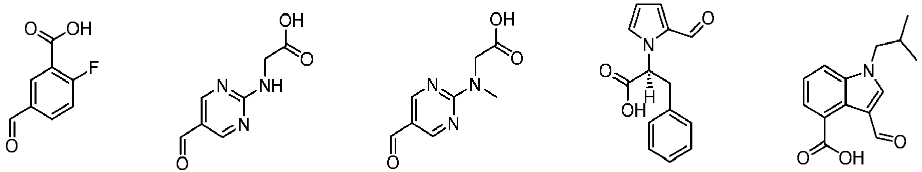

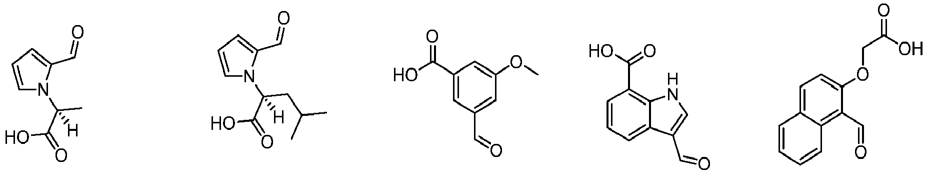

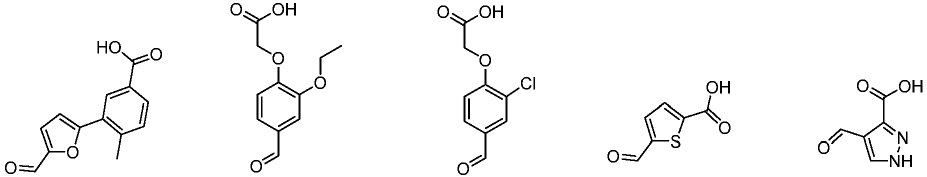

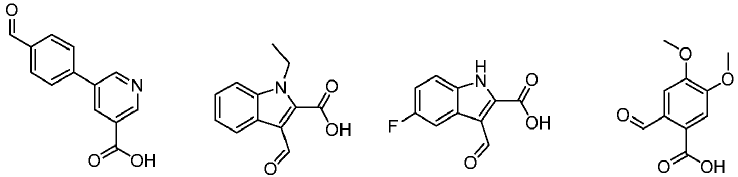

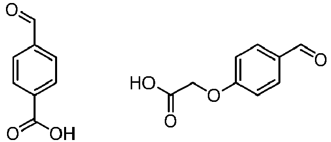

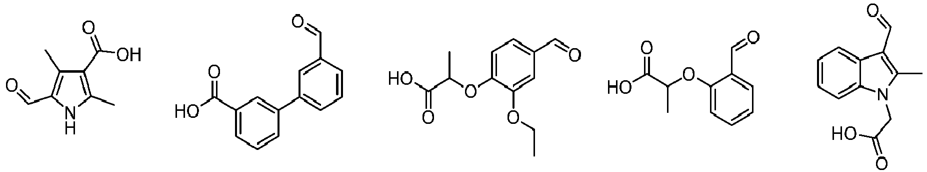

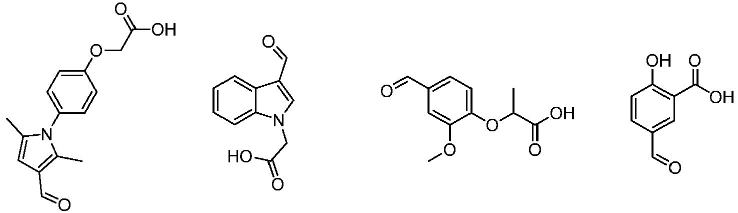

### Example 5: Barcoded compound synthesis by acid-mediated Povarov reaction

### (R)-(-)-BNDHP-mediated Povarov reaction on CPG-coupled code I

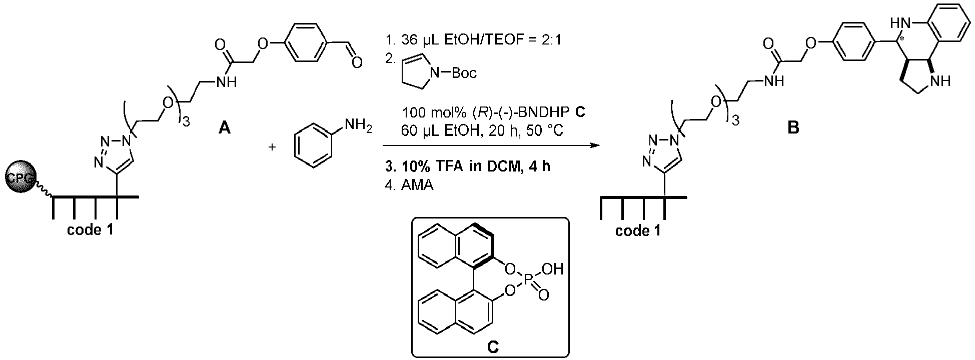

### Protocol:

Prior to use, CPG-coupled oligonucleotide, solid anilines, and (*R*)-(-)-BNDHP were dried *in vacuo* for 15 min. Aniline (10 µmol, 500 equiv.) was dissolved in 24 µL ethanol. The solution was added to CPG-coupled oligonucleotide-aldehyde conjugate **A** (20 nmol) suspended in 12 µL triethyl orthoformate. The suspension was shaken at ambient temperature for 4 h. Afterwards, 30 µL of (*R*)-(-)-BNDHP C (2 µmol, 100 equiv.) in ethanol followed by *N*-Boc-2,3-dihydro-1*H*-pyrrole (10 µmol, 500 equiv.) were added. The reaction mixture was shaken at 50 °C for 16 h. Then, the CPG-coupled oligonucleotide conjugate was filtered over a filter column, washed three times with each DMF, MeOH, CAN, and CH₂Cl₂ and dried *in vacuo.* The Boc-protecting group was removed by addition of 100 µL 10 % trifluoroacetic acid in CH₂Cl₂ for 4 h. Afterwards, CPG-coupled DNA was filtered over a filter column, washed with excess of 1 % TEA and three times with each 200 µL of DMF, MeOH, ACN and CH₂Cl₂ and dried *in vacuo.* CPG-coupled oligonucleotide conjugates **B** were cleaved from solid support and deprotected with 500 µL AMA (AMA = aqueous ammonia (30 %)/ aqueous methylamine (40 %), 1:1, vol/vol) for 4 hours at ambient temperature. Afterwards, 20 µL of 1 M Tris buffer (pH = 7.5) were added, the mixture was dried under reduced pressure (SpeedVac) and DNA was dissolved in 200 µL distilled water. The crude reaction mixture was analyzed by analytical RP-HPLC (Method I) and MALDI-TOF-MS. The product was purified by preparative RP-HPLC.

### Example 6: DNA amplification and sequencing experiments

Gel electrophoresis of two PCR experiments performed on chemically modified DNA templates. The templates were synthesized by DNA ligation of DNA barcode fragments, and the PCR was performed with primers used to read out DEL selection experiments. PCR amplification of templates consisting of 8-aza-7-deazaadenosine (5'-CT*CTCTTTA**A**CTA**CCT-3'; A** = 7-deaza-8-azaadenosine; T* = attachment point for the organic moiety) and 7-deazaadenosine (5'-CT*CTCTTTA*A*CTA*CCT-3'; A* = 7-deazaadenosine; T* = attachment point for the organic moiety) by Taq polymerase were compared head-to-head. PCR amplification products obtained from a template containing three 7-deaza-adenines and a template containing three 8-aza-7-deazaadenines were obtained at similar efficiencies. Even more important, a Sanger sequencing run proved high fidelity copying of both modified DNA strands. This result shows that DNA modified with 8-aza-7-deazaadenosines can be copied by PCR with standard nucleotide triphosphates. 8-aza-7-deazaadenosine positions at isolated positions or two in a row were read with the same fidelity as 7-deazaadenosine (data not shown). The experiment shown in Figure 4 was successfully repeated, i.e. similar results were obtained albeit not shown in Figure 4, with a template containing two 8-aza-7-deazaguanosines and a single 8-aza-7-deazaadenosine.

A standard PCR amplification reaction can be performed on a template that contains 8-aza-7-deazapurines. 8-aza-7-deazapurines appear as fully functional, chemically stable substitutes in DNA barcodes and show the same chemical stability as pyrimidine bases, and are read by Taq polymerase. 8-aza-7-deazapurines also show the same chemical stability as pyrimidine bases.

### Example 7: Design of a barcoded compound and a PCR amplicon

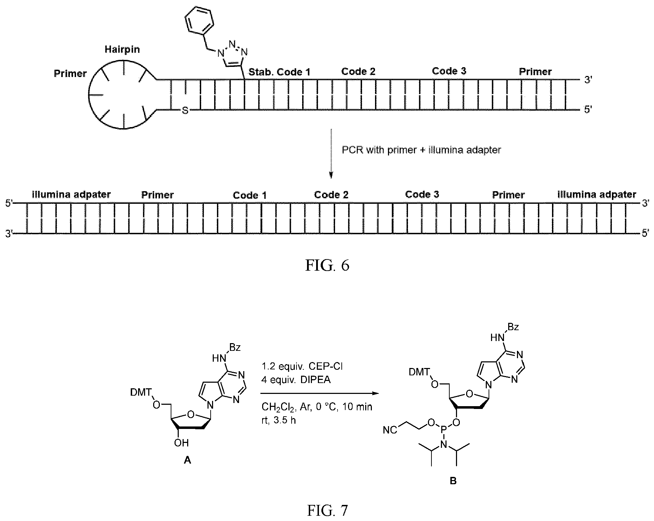

FIG. 7 shows an embodiment of a design of a final DNA-tagged compound and a PCR amplicon thereof. A DNA barcode included a linker within the sequence as opposed to at the 5'-terminal position of the sequence. Said differently, the linker was shifted from the 5'-terminal. FIG. 7 illustrates the encoding strategy using this position of the linker. Bifunctional starting materials that contain a carboxylic acid and a carbaldehyde (representative examples see Figure 6) were coupled to Code 1 through their carboxylic acid moiety, then pooled and reacted as pools to target compounds by a synthesis method discussed in Example 5 for a barcoded compound synthesis by acid-mediated Povarov reaction, above. The final compounds were cleaved from the solid phase, quality controlled, and ligated to a DNA hairpin containing the forward primer and a code 2 recording the heterocycle synthesis (Figure 3). Alternatively, the encoded library was finalized by a DNA-compatible coupling reaction and a further DNA ligation step according to Figure 4. The encoding strategy by ligation of chemically modified DNA barcode 1 (sequence II) to hairpin sequence I and barcode 2 was successfully validated and gave the data illustrated in the data, below.

This encoding strategy provides chemical diversity, can be scaled up, and provides fidelity when the more demanding chemistry, such as several heterocycle formation reactions, is performed earlier in the process. This encoding strategy provides flexibility, as e.g. DNA hairpin and codes 2 and 3 could also be composed of stable nucleobases if the chemistry done in the last step demands stable nucleobases.

### Coding of a two-cvcle DNA-encoded library

Hairpin DNA, coding oligonucleotides and ligation products were phosphorylated at the 5'-end with T4 polynucleotide kinase (T4 PNK, *Thermo Scientific Scientific*). The phosphorylation reactions were performed in a total volume of 20 µL using 280 pmol of the oligonucleotides, 10 units of T4 polynucleotide kinase (T4 PNK, *Thermo Fisher Scientific*), 1x PNK Buffer A (500 mM Tris-HCl, 100 mM MgCl₂, 50 mM DTT, 1 mM spermidine, pH = 7.6, 25 °C, *Thermo Fisher Scientific*) and 1 mM ATP (*Thermo Fisher Scientific*) were used. Reaction mixtures were incubated at 37 °C for 20 min, then heat-inactivated at 75 °C for 15 min and slowly cooled down to 4°C.

Prior to enzymatic ligation of DNA, the oligonucleotides were annealed by incubation at 85 °C for 10 min and cooling down to 4 °C. For ligation (20 µL scale), 100 pmol of each oligonucleotide, 600 units of T4 DNA Ligase (T4 DNA ligase rapid, *Biozym*) and 1x T4 DNA Ligase Buffer (500 mM Tris-HCl, 100 mM MgCl₂, 50 mM DTT, 10 mM ATP, pH = 7.6 at 25°C) were mixed. Ligation reactions were performed at 25 °C for 16 h, then stopped by heat inactivation at 75 °C for 15 min and cooled down to 4 °C.

For analysis of DNA ligation reactions, agarose gel electrophoresis was performed. Using a 5,5% agarose gel, electrophoresis was carried out in TBE buffer (89 mM Tris-borate, 2 mM EDTA, pH = 8.3) at 100 V constant voltage for 15 min and then 150 V constant voltage for about 45 min. For staining of the DNA, Midori Green Direct (*NIPPON Genetics*) and as a reference, GeneRuler Ultra Low Range DNA Ladder (*Thermo Fisher Scientific*) was used. Imaging of the gels was performed using the Bio-Rad Gel Doc™ XR system.

Table 2 shows the DNA sequences, which were used in the ligation process for a two-cycle library (Figure 2).

**Table 2: Generic sequences of the oligonucleotides I, II, II', III, and III'.**

| **DNA** | **length** | **sequence (5'-3')** |
|---|---|---|
| ***I*** | 37mer | CAA ATC CGT TCA S AGG TCG GTG TGA ACG GAT TTG AGT C (SEQ ID NO:4) |
| ***II*** | 16mer | CT*C TXX XXX XXX A*(or A**)CC T (SEQ ID NO:5); |
| | | X composed of C, T, A*, A**, G* |
| ***II'*** | 24mer | TAG G AG GTi aai iaa iAG AGG ACT (SEQ ID NO:6) |
| ***III*** | 35mer | CCT AGA CTT GAC TGA CCT CAA CTA CAT GGT CTA CA (SEQ ID NO:7) |
| ***III'*** | 31mer | TGT AGA CCA TGT AGT TGA GGT CAG TCA AGT C (SEQ ID NO:8) |

| | | |
|---|---|---|
| A* denotes 7-deaza adenosine; A** denotes 7-deaza-8-aza adenosine; G* denotes 7-deaza-8-aza guanosine; i denotes inosine; a denotes abasic site; T* denotes the attachment point for the organic moiety | | |

**Table 3: Functions of the partial sequences of oligonucleotides I, II, II, III and III'.**

| **partial sequence** | **function** | **length** | **sequence (5'-3')** |
|---|---|---|---|
| A | forward primer | 21mer | AGG TCG GTG TGA ACG GAT TTG (SEQ ID NO.9) |
| B | overhang for T4 DNA ligation | 4mer | AGTC |
| S | spacer (Taq DNA polymerase stopper) | - | triethylene glycol |
| B' | overhang for T4 DNA ligation | 4mer | GACT |
| C | code 1 | 8mer | XXXXXXXX composed of C, T, A*, A**, G* |
| C' | Universal counter strand to code 1 | 8mer | i aai iaa i |
| D | overhang for T4 DNA ligation | 4mer | CCTA |
| D' | overhang for T4 DNA ligation | 4mer | TAGG |
| E | code 2 | 8mer | XXX XXX XX |
| | | | X composed of C, T, A, G |
| E' | complementary sequence to code 2 | 8mer | XXX XXX XX |
| | | | X composed of C, T, A, G |
| F | reverse primer | 23mer | TGAC CTCA ACTA CATG GTCT ACA (SEQ ID NO:10) |
| F" | complementary sequence to reverse primer | 23mer | TGTA GACC ATGT AGTT GAGG TCA (SEQ ID NO:11) |

| | | | |
|---|---|---|---|
| A* denotes 7-deaza adenosine; A** denotes 7-deaza-8-aza adenosine; G* denotes 7-deaza-8-aza guanosine; i denotes inosine; a denotes abasic site. | | | |

**Table 4: Functions of the partial sequences of oligonucleotides I, II, II, III and III'.**

| **partial sequence** | **function** | **length** | **sequence (5'-3')** |
|---|---|---|---|
| A | forward primer | 21mer | AGG TCG GTG TGA ACG GAT TTG (SEQ ID NO:9) |
| B | overhang for T4 DNA ligation | 4mer | AGTC |
| S | spacer (Taq DNA polymerase blocker) | - | triethylene glycol |
| B' | overhang for T4 DNA ligation | 4mer | GACT |
| C | chemically modified DNA code (code 1) | 8mer | CTT TA*(A**, G*)A*(A**, G*) CT (part of SEQ ID Nos. 12-14) |
| C' | Universal sequence to chemically modified DNA code (code 1) | 8mer | i aai iaa i |
| D | overhang for T4 DNA ligation | 4mer | CCTA |
| D' | overhang for T4 DNA ligation | 4mer | TAGG |
| E | code 2 | 8mer | XXX XXX XX |
| | | | X composed of C, T, A, G |
| E' | complementary sequence to code 2 | 8mer | XXX XXX XX |
| | | | X composed of C, T, A, G |
| F | reverse primer | 23mer | TGAC CTCA ACTA CATG GTCT ACA (SEQ ID NO:10) |
| F" | complementary sequence to reverse primer | 23mer | TGTA GACC ATGT AGTT GAGG TCA (SEQ ID NO:11) |

| | | | |
|---|---|---|---|
| A* denotes 7-deaza adenosine; A** denotes 7-deaza-8-aza adenosine; G* denotes 7-deaza-8-aza guanosine; i denotes inosine; a denotes abasic site. | | | |

**Table 5: Sequences of exemplary oligonucleotides I, II, II', III, and III' (Figure 3).**

| **DNA** | **length** | **sequence (5'-3')** |
|---|---|---|
| ***I*** | 37mer | CAA ATC CGT TCA S AGG TCG GTG TGA ACG GAT TTG AGT C (SEQ ID NO:4) |
| ***II*** | 16mer | CT*C TCT TTA* A*CT A*CC T (code A) (SEQ ID NO: 12) |
| | | CT*C TCT TTA** A**CT A**CC T (code B) (SEQ ID NO: 13) |
| | | CT*C TCT TTG* G*CT A**CC T (code C) (SEQ ID NO: 14) |
| ***II'*** | 24mer | TAG GAG GTi aai iaa iAG AGG ACT (SEQ ID NO:6) |
| ***III*** | 35mer | CCT AGA CTT GAC TGA CCT CAA CTA CAT GGT CTA CA (SEQ ID NO:7) |
| ***III**'* | 31mer | TGT AGA CCA TGT AGT TGA GGT CAG TCA AGT C (SEQ ID NO:8) |

| | | |
|---|---|---|
| A* denotes 7-deaza adenosine; A** denotes 7-deaza-8-aza adenosine; G* denotes 7-deaza-8-aza guanosine; i denotes inosine; a denotes abasic site; T* denotes the attachment point for the organic moiety. | | |

### Example 8: PCR amplification of chemically modified nucleic acids

Ligation reaction of the hairpin DNA I with the chemically modified duplex DNA ***II*/*II***' and native duplex DNA ***III***/***III***' (as set forth in Table 5). The following reactions were carried out and analyzed by gel electrophoresis: 1: one-pot ligation of hairpin DNA ***I*** with DNA duplex ***II***/***II***' and DNA duplex ***III***/***III***'; 2: ligation of hairpin DNA ***I*** with DNA duplex ***II***/***II***', 7-deaza-8-aza adenosine was used. The results show that in all reactions carried out the ligation product was obtained (data not shown).

DNA obtained by enzymatic ligation of hairpin DNA ***I*** to chemically modified duplex DNA ***II***/***II***' and duplex DNA ***III*/*III'*** (Figure 1-4, Table 4) were amplified by PCR (forward primer (54mer): 5'-TCG TCG GCA GCG TCA GAT GTG TAT AAG AGA CAG AGG TCG GTG TGA ACG GAT TTG-3' (SEQ ID NO: 15), reverse primer (57mer): 5'-GTC TCG TGG GCT CGG AGA TGT GTA TAA GAG ACA GTG TAG ACC ATG TAG TTG AGG TCA-3' (SEQ ID NO: 16).

For PCR amplification (40 µL scale), 10 µL of ligation product, 1 µM of reverse primer, 5 units of Taq DNA polymerase (*Thermo Fisher Scientific),* 1x Taq DNA Polymerase Buffer (100 mM Tris-HCl, pH 8.8 at 25 °C, 500 mM KCl, 0.8 % (v/v) Nonidet P40), 3 mM MgCl₂ and 0.625 mM of each dNTP (dATP, dGTP, dTTP, and dCTP, corresponding to 2.5 mM of the mixture of dNTPs) were mixed. The PCR program started with pre-denaturation at 95 °C for 3 min, followed by denaturation for 30 s at 95 °C, annealing for 30 s at 55 °C, and elongation for 30 s at 72 °C. After 10 PCR cycles, 1 µM of forward primer were added and additional 20 PCR cycles were performed.

For analysis of DNA amplification reactions, agarose gel electrophoresis was performed. Using a 5,5% agarose gel, electrophoresis was carried out in TBE buffer (89 mM Tris-borate, 2 mM EDTA, pH = 8.3) at 100 V constant voltage for 15 min and then 150 V constant voltage for about 45 min. For staining of the DNA, Midori Green Direct (*NIPPON Genetics*) and as a reference, GeneRuler Ultra Low Range DNA Ladder (*Thermo Fisher Scientific*) was used. Imaging of the gels was performed using the Bio-Rad Gel Doc™ XR system.

Gel analysis of a PCR amplification of chemically modified DNA ligation product. 1: PCR product of DNA ligation product containing DNA hairpin ***I***, DNA duplex ***II*/*II'*** and DNA duplex ***III*/*III'*;** 2: one-pot ligation of hairpin DNA ***I*** with DNA duplex ***II*/*II'*** and DNA duplex ***III*/*III'*;** 3: ligation of hairpin DNA ***I*** with DNA duplex ***II*/*II'*;** 7-deaza adenosine was used. The results show that in all reactions carried out the ligation product was obtained (data not shown).

PCR amplification of chemically modified DNA ligation product. 1: DNA hairpin ***I***; 2: one-pot ligation of hairpin DNA ***I*** with DNA duplex ***II*/*II*'** and DNA duplex ***III*/*III*'**; 3: PCR product of DNA ligation product containing DNA hairpin ***I***, DNA duplex ***II*/*II*'** and DNA duplex ***III*/*III*'.** 7-deaza-8-aza adenosine was used. The results show that in all reactions carried out the ligation product was obtained (data not shown).

### Example 9: Sequencing of chemically modified nucleic acids

DNA obtained by PCR amplification were purified using the QIAquick PCR Purification Kit (*QIAGEN*) according to the protocol. Sequencing of the DNA was done by *GATC.*

The sequencing data was aligned against the template DNA synthesized from chemically modified code A, code B or code C as shown in Table 5. The data showed the expected sequence (data not shown).

### Example 10: Synthesis of a three-cycle library

**Table 6: Sequences of the oligonucleotides I, II, II', III, III'; IV, and IV.**

| **DNA** | **length** | **sequence (5'-3')** |
|---|---|---|
| ***I*** | 38mer | CAAA TCCG TTCA SAGG TCGG TGTG AACG GATT TGAG TC (SEQ ID NO:4) |
| ***II*** | 16mer | CT*C TXX XXX XXX A*(or A**)CC T; |
| | | X composed of C, T, A*, A**, G* (SEQ ID NO:5) |
| ***II'*** | 24mer | ATA CAGG T i aai iaa iAG AGG ACT (SEQ ID NO:6) |
| ***III*** | 12mer | XXX XXX XXA CTC (SEQ ID NO:18) |
| | | X composed of C, T, A, G |
| ***III'*** | 12mer | TAG GXX XXX XXX (SEQ ID NO:19) |
| | | X composed of C, T, A, G |
| ***IV*** | 35mer | CCT AXX XXX XXX TGA CCT CAA CTA CAT GGT CTA CA (SEQ ID NO:20); X composed of C, T, A, G |
| ***IV'*** | 31mer | XXX XXX XXTGT AGTT GAGG TCAA TCGT TCG (SEQ ID NO:21); X composed of C, T, A, G |

| | | |
|---|---|---|
| A* denotes 7-deaza adenosine; A** denotes 7-deaza-8-aza adenosine; G* denotes 7-deaza-8-aza guanosine; i denotes inosine; a denotes abasic site; T* denotes the attachment point for the organic moiety. | | |

**Table 7: Functions of the partial sequences of oligonucleotides I, II, II', III, III', IV, and IV'.**

| **sequence** | **Function** | **length** | **sequence (5'-3')** |
|---|---|---|---|
| A | forward primer | 21mer | AGGT CGGT GTGA ACGG ATT TG (SEQ ID NO:9) |
| B | overhang for T4 DNA ligation | 4mer | AGTC |
| S | spacer (Taq DNA polymerase stopper) | 1 | triethylene glycol |
| B' | overhang for T4 DNA ligation | 4mer | GACT |
| C | code 1 | 8mer | XXXXXXXX |
| | | | X composed of C, T, A*, A**, G* |
| C' | degenerate sequence annealed to code 1 | 8mer | i aai iaa i |
| D | overhang for T4 DNA | 4mer | GTAT |
| D' | overhang for T4 DNA | 4mer | ATAC |
| E | code 2 | 8mer | XXX XXX XX |
| | | | X composed of C, T, A, G |
| E' | complementary sequence to code 2 | 8mer | XXX XXX XX |
| | | | X composed of C, T, A, G |
| F | overhang for T4 DNA | 4mer | CCTA |
| F' | complementary sequence to overhang for T4 DNA | 4mer | TAGG |
| G | code 3 | 8mer | XXXXXXXX |
| | | | X composed of C, T, A, G |
| G' | complementary sequence to code 3 | 8mer | XXX XXX XX |
| | | | X composed of C, T, A, G |
| H | reverse primer | 23mer | TGAC CTCA ACTA CATG GTCT ACA; (SEQ ID NO:10) |
| H' | complementary sequence to reverse primer | 23mer | TGTA GACC ATGT AGTT GAGG TCA; (SEQ ID NO:11) |

| | | | |
|---|---|---|---|
| A* denotes 7-deaza adenosine; A** denotes 7-deaza-8-aza adenosine; G* denotes 7-deaza-8-aza guanosine; i denotes inosine; a denotes abasic site. | | | |

**Table 8: Exemplary sequences of the oligonucleotide codes II/II'.**

| **DNA** | **sequence** |
|---|---|
| code | (***II*/*****II**'*)5'-3' |
| ***II*/*****II**'*₁ | CTCT CATG GATC ACCT (SEQ ID NO:22) |
| | ATA CAGG T i aai iaa iAG AGG ACT (SEQ ID NO:23) |
| ***II*/*II'*₂** | CTCT CATG G**A**TC ACCT (SEQ ID NO:24) |
| | ATA CAGG T i aai iaa iAG AGG ACT (SEQ ID NO:23) |
| ***II*/*****II**'*₃ | CTCT CATG** G**ATC ACCT (SEQ ID NO:25) |
| | ATA CAGG T i aai iaa iAG AGG ACT (SEQ ID NO:23) |
| ***II*/*****II**'*₄ | CTCT CA**TG GA**TC ACCT (SEQ ID NO:26) |
| | ATA CAGG T i aai iaa iAG AGG ACT (SEQ ID NO:23) |
| ***II*/*****II**'*₅ | CTCT CATG** G**ATC ACCT (SEQ ID NO:25) |
| | ATA CAGG T i aai iaa iAG AGG ACT (SEQ ID NO:23) |
| ***II*/*****II**'*₆ | CTCT CATG GATC ACCT (SEQ ID NO:22) |
| | ATA CAGG T i aai iaa iAG AGG ACT (SEQ ID NO:23) |
| ***II*/*****II**'*₇ | CTCT CATG G**A**TC ACCT (SEQ ID NO:24) |
| | ATA CAGG T i aai iaa iAG AGG ACT (SEQ ID NO:23) |
| ***II*/*****II**'*₈ | CTCT CA**TG GA**TC ACCT (SEQ ID NO:26) |
| | ATA CAGG T i aai iaa iAG AGG ACT (SEQ ID NO:23) |
| ***II*/*****II**'*₉ | CTCT CATG G**A**TC ACCT (SEQ ID NO:24) |
| | ATA CAGG T i aai iaa iAG AGG ACT (SEQ ID NO:23) |
| ***II*/*****II**'*₁₀ | CTCT CATG** G**ATC ACCT (SEQ ID NO:25) |
| | ATA CAGG T i aai iaa iAG AGG ACT (SEQ ID NO:23) |
| ***II*/*****II**'*₁₁ | CTCT CA**TG GA**TC ACCT (SEQ ID NO:26) |
| | ATA CAGG T i aai iaa iAG AGG ACT (SEQ ID NO:23) |
| ***II*/*****II**'*₁₂ | CTCT CATG GATC ACCT (SEQ ID NO:22) |
| | ATA CAGG T i aai iaa iAG AGG ACT (SEQ ID NO:23) |

| | |
|---|---|
| A** denotes 7-deaza-8-azaadenosine; G** denotes 7-deaza-8-azaguanosine. | |

### Protocol:

Hairpin DNA and coding oligonucleotides were phosphorylated at the 5'-end with T4 polynucleotide kinase (T4 PNK, *Thermo Scientific Scientific*). The phosphorylation reactions were performed in a total volume of 20 µl using 280 pmol of the coding oligonucleotides (e.g. oligonucleotides ***I***, ***II***, ***II'***, ***III***, ***III*'***, **IV*** (Figure 1)).

**Table 9: reaction mixture of phosphorylation reaction with PNK for the one-pot ligation.**

| | |
|---|---|
| oligonucleotide ***I*** or ***II*** or ***II***' or ***III*** or ***III'*** or ***IV*** | 280 pmol |
| 1X PNK buffer A (500 mM Tris-HCl; pH 7.6 at 25 °C, 100 mM MgCl₂, 50 mM DTT, 1 mM spermidine, *Thermo Scientific Scientific*) | 2 µl |
| ATP (1 mM, *Thermo Scientific Scientific*) | 2 µl |
| T4 Polynucleotide Kinase | 1 µl (10 u) |
| water, nuclease-free | to 20 µl |

The PNK reaction mixtures were incubated at 37 °C for 20 minutes. The inactivation of PNK was done by incubating the samples at 75 °C for 15 minutes and then cooling them down slowly to 4 °C.

Prior to ligation, 20 pmol of each oligonucleotide: *I* (phosphorylated), ***II*** (phosphorylated), ***II*'** (phosphorylated), ***III*** (phosphorylated), ***III'*** (phosphorylated), ***IV'*** (phosphorylated), and **IV** were pipetted together and 1x T4 DNA ligase buffer (500 mM Tris-HCI, 100 mM MgCl₂, 50 mM DTT, 10 mM ATP, pH 7.6 at 25 °C, *Biozym*) was added. The reaction mixture was filled with distilled water to reach the total volume of 19 µl. Then, oligonucleotides were annealed at 85 °C for 10 minutes and after that slowly cooled down to 4 °C.

### Ligation protocol:

For ligation reactions 1 µL of T4 DNA ligase (600 U/µl, T4 DNA ligase rapid, *Biozym*) was added to annealed oligonucleotides for a total reaction volume of 20 µl. The reaction mixtures were incubated at 25 °C for 16 hours. T4 DNA ligase was inactivated by incubating the samples at 75 °C for 15 minutes, followed by slow cool down to 4 °C.

To confirm the ligation process, agarose gel (5.5% agarose) electrophoresis was used to identify the components in the ligation reaction mixtures, shown in Figure 2. Electrophoresis was carried out in 1 x TBE buffer (0.1 M Tris, 0.1 M H₃BO₃, 0.2 mM EDTA, pH= 8.3) at 100 voltage (V) for 10 min and then at 150 V for 50-55 min. DNA was stained with Midori Green Direct, and the GeneRuler Ultra Low Range DNA Ladder was used as reference.

Gel analysis of the one-pot ligation reaction of hairpin DNA ***I*** with the 12 DNA codes *I****I*/*****II**_{'1-12}* (Table 4), native duplex DNA code ***III*/*III*'**, and native duplex DNA code ***IV*/*IV'*.** 1: DNA hairpin ***I***; 2: product of the ligation of the hairpin ***I*** with code ***II*/*II'₁***; 3: product of the one-pot ligation of the hairpin ***I*** with code ***II*/*****II'₁**,* code ***III*/*III'***, and code ***IVlV*'**; 4: product of the one-pot ligation of the hairpin ***I*** with code ***II*/*****II'**₂,* code ***III*/*****III**,* and code ***IV*/*IV'***; 5: product of the one-pot ligation of the hairpin ***I*** with code ***II*/*****II**'₃*, code ***III*/*III'***, and code ***IV*/*IV*'**; 6: product of the one-pot ligation of the hairpin ***I*** with code ***II*/*****II'**₄*, code ***III*/*III'***, and code ***IV*/*IV'***; 7: product of the one-pot ligation of the hairpin ***I*** with code ***II*/*****II**'₅,* code ***III*/*III'***, and code ***IV*/*IV'***; 8: product of the one-pot ligation of the hairpin ***I*** with code ***II*/*****II**'₆*, code ***III*/*III'***, and code ***IV*/*****IV**'*; 9: product of the one-pot ligation of the hairpin ***I*** with code ***II*/*****II**'₇*, code ***III*/*****III**',* and code ***IV*/*IV'***; 10: product of the one-pot ligation of the hairpin ***I*** with code ***II*/*II'***8*,* code ***III*/*****III'**,* and code ***IV*/*****IV**'*; 11: product of the one-pot ligation of the hairpin ***I*** with code ***II*/*****II**'₉,* code ***III***/***III**'*, and code ***IV***/***IV'***; 12: product of the one-pot ligation of the hairpin ***I*** with code ***II*/*****II'**₁₀*, code ***III*/*****III**'*, and code ***IV*/*IV'***: 13: product of the one-pot ligation of the hairpin ***I*** with code ***II*/*****II'**₁₁*, code ***III*/*****III**',* and code ***IV*/*IV'***. 14: product of the one-pot ligation of the hairpin ***I*** with code ***II*/*****II**'₁₂,* code ***III*/*****III'**,* and code ***IV*/*IV'***. The results show that in all reactions carried out the expected ligation product was obtained (data not shown).

### PCR amplification of chemically modified nucleic acids

1 amol of 81 mer DNA templates obtained by one-pot ligation of hairpin DNA ***I*** to duplex DNA ***II*/*****II**'*, duplex DNA ***III*/*****III**'*, and duplex DNA ***IV*/*IV'*** (Figure 1 and 2, Table 4) was amplified by PCR with Illumina adapters (forward primer (54mer): 5'-TCG TCG GCA GCG TCA GAT GTG TAT AAG AGA CAG AGG TCG GTG TGA ACG GAT TTG-3', reverse primer (57mer): 5'-GTC TCG TGG GCT CGG AGA TGT GTA TAA GAG ACA GTG TAG ACC ATG TAG TTG AGG TCA-3').

### PCR protocol

The final volume of PCR reaction was 40 µL and contained 4 µL of 10 x PCR Rxn buffer, 1 µM of reverse primer, 3 mM of MgCl₂, 0.625 mM of each dNTP (dATP, dGTP, dTTP, and dCTP, corresponding to 2.5 mM of the mixture of dNTPs), and 10 µL of the template 81mer DNA (one-pot ligation product). For amplification of the 81mer DNA template five units (1 µL) of Taq DNA polymerase were used. The PCR program started with pre-denaturation at 95 °C for three minutes, followed by denaturation for 30 seconds at 95 °C, annealing for 30 seconds at 55 °C, and elongation for 30 seconds at 72 °C. After 10 PCR cycles, 1 µM of forward primer was added, and additional 20 PCR cycles were performed.

PCR reactions were analyzed by agarose gel electrophoresis (5.5% agarose). Electrophoresis was carried out in 1 x TBE buffer (0.1 M Tris, 0.1 M H₃BO₃, 0.2 mM EDTA, pH= 8.3) at 100 voltage (V) for 10 min and then at 150 V for 50-55 min. DNA was stained with Midori Green Direct, and the GeneRuler Ultra Low Range DNA Ladder was used as reference.

PCR amplification of 81mer DNA ligation products was analyzed by gel electrophoresis. 1: 5 pmol of the product (81mer) of the one-pot ligation of the hairpin ***I*** with code ***II*/*****II**'₇*, code ***III*/*III'***, and code ***IV*/*IV'*** ; 2: 1 amol of the product of the one-pot ligation of the hairpin ***I*** with code ***II*/*****II'**₇*, code ***III*/*****III**'*, and code ***IV*/*IV'*** ; 3: product of the PCR amplification of 1 amol of 81mer DNA template containing code ***II*/*****II**'₁*; 4: product of the PCR amplification of 1 amol of 81mer DNA template containing code ***II*/*****II'**₂*; 5: product of the PCR amplification of 1 amol of 81mer DNA template containing code ***II*/*****II**'₇*; 6: product of the PCR amplification of 1 amol of 81mer DNA template containing code ***II*/*II'₅***; 7: product of the PCR amplification of 1 amol of 81 mer DNA template containing code ***II*/*****II**'**₈***; 8: product of the PCR amplification of 1 amol of 81mer DNA template containing code ***II*/*****II'***_{*12*;} 9: negative control experiment (Taq DNA polymerase omitted); 10: product of the PCR amplification of 1 amol of 81mer DNA template containing code ***II*/*****II'**₁₀*; 11: product of the PCR amplification of 1 amol of 81mer DNA template containing code ***II*/*****II'**₁₁.* The results show that in all reactions carried out the expected ligation product was obtained (data not shown).

All documents cited herein, are hereby incorporated by reference in their entirety.

The inventions illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the inventions embodied therein herein disclosed may be resorted to by those skilled in the art, and that such modifications and variations are considered to be within the scope of this invention. The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein. In addition, where features or aspects of the invention are described in terms of Markush groups, those skilled in the art will recognize that the invention is also thereby described in terms of any individual member or subgroup of members of the Markush group. Further embodiments of the invention will become apparent from the following claims.

## Claims

1. A compound library comprising a plurality of conjugate molecules, wherein each conjugate molecule comprises a small organic molecule covalently coupled to a nucleic acid moiety, wherein the nucleic acid moiety comprises or consists of 7-deazapurines and/or 7-deaza-8-azapurines, and optionally modified and/or unmodified pyrimidine nucleotides.

2. The compound library according to claim 1, wherein the 7-deazapurines and/or 7-deaza-8-azapurines are selected from 7-deaza-8-azaadenosine, 7-deaza-8-azaguanosine, or combinations thereof.

3. The compound library according to claim 1 or 2, wherein the 7-deazapurines and/or 7-deaza-8-azapurines comprises 7-aza- or 7-deaza-8-aza-modified inosine, 7-aza- or 7-deaza-8-aza-modified N⁶-methyladenosine, 7-aza- or 7-deaza-8-aza-modified xanthosine, or combinations thereof.

4. The compound library according to any of the preceding claims, wherein the plurality of conjugate molecules comprises at least ten conjugate molecules, preferably at least 50 conjugate molecules, more preferably at least 100 conjugate molecules.

5. The compound library according to any of the preceding claims, wherein the nucleic acid moiety comprises at least 2 nucleotides, preferably at least 8 or 10 nucleotides.

6. The compound library according to any of the preceding claims, wherein the nucleic acid moiety is RNA, DNA, or a hybrid thereof.

7. The compound library according to any of the preceding claims, wherein
a) the conjugate molecules further comprise a linker between the nucleic acid moiety and the organic molecule, preferably the linker is polyethylene glycol (PEG);
b) the conjugate molecules differ from each other by comprising different nucleic acid moieties;
c) the organic molecule consists of 2 or more carbon atoms, preferably up to 50 carbon atoms, more preferably up to 30 carbon atoms; and/or
d) the organic molecule has a molecular weight of at most 900 daltons, preferably at most 700 daltons and more preferably at most 500 daltons.

8. A method for synthesizing a compound library, wherein said library comprises a plurality of conjugate molecules, wherein said conjugates comprise a small organic molecule covalently coupled to a nucleic acid moiety, the synthesis of each conjugate molecule comprises:
- reacting a nucleic acid consisting of 7-deazapurines and/or 7-deaza-8-azapurines and, optionally modified and/or unmodified pyrimidine nucleotides, with an organic molecule under conditions to allow the conjugation of said molecules; and, optionally,
- subjecting the conjugate obtained in step (1) to reaction conditions that allow modification of the small organic molecule; and, optionally,
- elongating the nucleic acid moiety of the conjugate obtained in step (2) by adding a further nucleic acid sequence.

9. The method according to claim 8, wherein the 7-deazapurines and/or 7-deaza-8-azapurines are selected from 7-deaza-8-azaadenosine, 7-deaza-8-azaguanosine, or combinations thereof.

10. The method according to claim 8 or 9, wherein the 7-deazapurines and/or 7-deaza-8-azapurines comprises 7-aza- or 7-deaza-8-aza-modified inosine, 7-aza- or 7-deaza-8-aza-modified N⁶-methyladenosine, 7-aza- or 7-deaza-8-aza-modified xanthosine, or combinations thereof.

11. The method according to claims 8-10, wherein reacting the nucleic acid with the organic molecule and subjecting the conjugate obtained to reaction conditions that allow modification of the small organic molecule comprise:
a) reacting the nucleic acid with a first organic intermediate molecule under conditions to form a conjugate of the nucleic acid and the first organic intermediate molecule; and
b) reacting the first organic intermediate molecule of the conjugate with a second organic intermediate molecule to form a conjugate molecule of the nucleic acid and the reaction product of the first and second organic intermediate molecules.

12. The method according to claims 8-11, the elongation step further comprising:
a) phosphorylating a 5'-terminal end of a sequence of the nucleic acid moiety; and
b) ligating a further nucleic acid sequence by a T4 ligase.

13. The method according to any one of claims 8-12, wherein the nucleic acid moiety in the first reaction step
a) consists of two to 20 nucleotides, preferably three to twelve nucleotides;
b) is RNA, DNA or a mixture thereof, preferably DNA;
c) is elongated in an additional reaction step with modified and/or unmodified pyrimidine and/or purine nucleotides in the elongation step; and/or
d) combinations thereof.

14. The method according to any one of claims 8-13, wherein
a) the conjugates further comprise a linker between the nucleic acid moiety and the organic molecule, preferably this linker portion is polyethylene glycol (PEG);
b) the conjugates differ from each other by comprising different nucleic acid moieties;
c) the organic molecule consists of 2 or more carbon atoms, preferably up to 50 carbon atoms, more preferably up to 30 carbon atoms;
d) the organic molecule has a molecular weight of at most 900 daltons, preferably at most 700 daltons and more preferably at most 500 daltons; and/or
e) combinations thereof.

15. Use of a compound library according to any one of claims 1 to 7 for screening a compound binding to a target molecule, preferably the target molecule is a protein.


SEQUENCE LISTING

<110>  Technische Universität Dortmund

<120>  DNA-ENCODED CHEMICAL LIBRARY, USE THEREOF, AND METHOD TO
       SYNTHESIZE THE LIBRARY

<130>  P76166

<160>  26

<170>  PatentIn version 3.5

<210>  1
<211>  10
<212>  DNA
<213>  Artificial  sequence

<220>
<223>  5′ - 3′ sequence of ATC

<400>  1
ttactaccta                                                              10


<210>  2
<211>  10
<212>  DNA
<213>  Artificial sequence

<220>
<223>  5′ - 3′ sequence of A*TC


<220>
<221>  misc_feature
<222>  (3)..(3)
<223>  n is 7-deaza-2′-deoxyadenosine

<220>
<221>  misc_feature
<222>  (6)..(6)
<223>  n is 7-deaza-2′-deoxyadenosine

<220>
<221>  misc_feature
<222>  (9)..(9)
<223>  n is 7-deaza-2′-deoxyadenosine

<400>  2
ttnctnccnt                                                              10


<210>  3
<211>  10
<212>  DNA
<213>  Artificial sequence

<220>
<223>  5′ - 3′ sequence of A**TC


<220>

<221>  misc_feature
<222>  (3)..(3)
<223>  n is 7-deaza-8-aza-2′deoxyadenosine

<220>
<221>  misc_feature
<222>  (6)..(6)
<223>  n is 7-deaza-8-aza-2′deoxyadenosine

<220>
<221>  misc_feature
<222>  (9)..(9)
<223>  n is 7-deaza-8-aza-2′deoxyadenosine

<400>  3
ttnctnccnt                                                              10


<210>  4
<211>  38
<212>  DNA
<213>  Artificial sequcence

<220>
<223>  Generic sequence of the oligonucleotide I


<220>
<221>  misc_feature
<222>  (13)..(13)
<223>  n is triethylene glycol

<220>
<221>  misc_feature
<222>  (13)..(13)
<223>  n is triethylene glycol (spacer)

<400>  4
caaatccgtt canaggtcgg tgtgaacgga tttgagtc                               38


<210>  5
<211>  16
<212>  DNA
<213>  Artificial sequence

<220>
<223>  Generic sequence of the oligonucleotide II


<220>
<221>  misc_feature
<222>  (2)..(2)
<223>  n is 7-deaza-8-aza thymidine

<220>
<221>  misc_feature
<222>  (5)..(12)
<223>  each n is independently of each other C, T, 7-deaza adenosine,
       7-deaza-8-aza adenosine or 7-deaza-8-aza guanosine

<220>
<221>  misc_feature

<222>  (13)..(13)
<223>  n is 7-deaza adenosine or 7-deaza-8-aza adenosine

<400>  5
cnctnnnnnn nnncct                                                    16


<210>  6
<211>  24
<212>  DNA
<213>  Artificial sequence

<220>
<223>  Generic sequence of the oligonucleotide II'


<220>
<221>  misc_feature
<222>  (9)..(9)
<223>  n is inosine

<220>
<221>  misc_feature
<222>  (10)..(10)
<223>  n is an abasic site

<220>
<221>  misc_feature
<222>  (11)..(11)
<223>  n is an abasic site

<220>
<221>  misc_feature
<222>  (12)..(12)
<223>  n is inosine

<220>
<221>  misc_feature
<222>  (13)..(13)
<223>  n is an abasic site

<220>
<221>  misc_feature
<222>  (14)..(14)
<223>  n is an abasic site

<220>
<221>  misc_feature
<222>  (15)..(15)
<223>  n is inosine

<220>
<221>  misc_feature
<222>  (16)..(16)
<223>  n is inosine

<400>  6
   taggaggtnn nnnnnnagag gact                                            24


<210>  7
<211>  35
<212>  DNA

<213>  Artificial sequence

<220>
<223>  Generic sequence of the oligonucleotide III

<400>  7
cctagacttg actgacctca actacatggt ctaca                                35


<210>  8
<211>  31
<212>  DNA
<213>  Artificial sequence

<220>
<223>  Generic sequence of the olionucleotide III`

<400>  8
tgtagaccat gtagttgagg tcagtcaagt c                                    31


<210>  9
<211>  21
<212>  DNA
<213>  Artificial sequence

<220>
<223>  Forward primer

<400>  9
aggtcggtgt gaacggattt g                                               21


<210>  10
<211>  23
<212>  DNA
<213>  Artificial sequence

<220>
<223>  Reverse primer

<400>  10
tgacctcaac tacatggtct aca                                             23


<210>  11
<211>  23
<212>  DNA
<213>  Artificial sequence

<220>
<223>  Complementary sequence to reverse primer

<400>  11
tgtagaccat gtagttgagg tca                                             23


<210>  12
<211>  16
<212>  DNA
<213>  Artificial sequence

<220>

<223>  Sequence of exemplary oligonucleotide II (code A)

<220>
<221>  misc_feature
<222>  (2)..(2)
<223>  n is T

<220>
<221>  misc_feature
<222>  (9)..(9)
<223>  n is 7-deaza adenosine

<220>
<221>  misc_feature
<222>  (10)..(10)
<223>  n is 7-deaza adenosine

<220>
<221>  misc_feature
<222>  (13)..(13)
<223>  n is 7-deaza adenosine

<400>  12
cnctctttnn ctncct                                                    16

<210>  13
<211>  16
<212>  DNA
<213>  Artificial sequence

<220>
<223>  Sequence of exemplary oligonucleotide II (code B)

<220>
<221>  misc_feature
<222>  (2)..(2)
<223>  n is T

<220>
<221>  misc_feature
<222>  (9)..(9)
<223>  n is 7-deaza-8-aza adenosine

<220>
<221>  misc_feature
<222>  (10)..(10)
<223>  n is 7-deaza-8-aza adenosine

<220>
<221>  misc_feature
<222>  (13)..(13)
<223>  n is 7-deaza-8-aza adenosine

<400>  13
cnctctttnn ctncct                                                    16

<210>  14
<211>  16
<212>  DNA

<213>  Artificial sequence

<220>
<223>  Sequence of exemplary oligonucleotide II (code C)



<220>
<221>  misc_feature
<222>  (2)..(2)
<223>  n is T

<220>
<221>  misc_feature
<222>  (9)..(9)
<223>  n is 7-deaza-8-aza guanosine

<220>
<221>  misc_feature
<222>  (10)..(10)
<223>  n is 7-deaza-8-aza guanosine

<220>
<221>  misc_feature
<222>  (13)..(13)
<223>  n is 7-deaza-8-aza adenosine

<400>  14
cnctctttnn ctncct                                                    16


<210>  15
<211>  54
<212>  DNA
<213>  Artificial sequence

<220>
<223>  Forward primer

<400>  15
tcgtcggcag cgtcagatgt gtataagaga cagaggtcgg tgtgaacgga tttg          54


<210>  16
<211>  57
<212>  DNA
<213>  Artificial sequence

<220>
<223>  Reverse primer

<400>  16
gtctcgtggg ctcggagatg tgtataagag acagtgtaga ccatgtagtt gaggtca       57


<210>  17
<211>  24
<212>  DNA
<213>  Artificial sequence

<220>
<223>  Sequence of the oligonucleotide II`

<220>
<221>  misc_feature
<222>  (9)..(9)
<223>  n is inosine

<220>
<221>  misc_feature
<222>  (10)..(10)
<223>  n is an abasic site

<220>
<221>  misc_feature
<222>  (11)..(11)
<223>  n is an abasic site

<220>
<221>  misc_feature
<222>  (12)..(12)
<223>  n is inosine

<220>
<221>  misc_feature
<222>  (13)..(13)
<223>  n is inosine

<220>
<221>  misc_feature
<222>  (14)..(14)
<223>  n is an abasic site

<220>
<221>  misc_feature
<222>  (15)..(15)
<223>  n is an abasic site

<220>
<221>  misc_feature
<222>  (16)..(16)
<223>  n is inosine

<400>  17
atacaggtnn nnnnnnagag gact                                              24


<210>  18
<211>  12
<212>  DNA
<213>  Artificial sequence

<220>
<223>  Sequence of the oligonucleotide III


<220>
<221>  misc_feature
<222>  (1)..(8)
<223>  each n is independently of each other C, T, A or G

<400>  18
nnnnnnnnac tc                                                           12


<210>  19

<211>  12
<212>  DNA
<213>  Artificial sequence

<220>
<223>  Sequence of the oligonucleotide III`


<220>
<221>  misc_feature
<222>  (5)..(12)
<223>  each n is independently of each other C, T, A or G

<400>  19
taggnnnnnn nn                                                          12


<210>  20
<211>  35
<212>  DNA
<213>  Artificial sequence

<220>
<223>  Sequence of the oligonucleotide IV


<220>
<221>  misc_feature
<222>  (5)..(12)
<223>  each n is independently of each other C, T, A or G

<400>  20
cctannnnnn nntgacctca actacatggt ctaca                                 35


<210>  21
<211>  30
<212>  DNA
<213>  Artificial sequence

<220>
<223>  Sequence of the oligonucleotide IV`


<220>
<221>  misc_feature
<222>  (1)..(8)
<223>  each n is independently of each other C, T, A or G

<400>  21
nnnnnnnntg tagttgaggt caatcgttcg                                       30


<210>  22
<211>  16
<212>  DNA
<213>  Artificial sequence

<220>
<223>  Exemplary sequence of the oligonucleotide code II

<400>  22
ctctcatgga tcacct                                                      16

<210>  23
<211>  24
<212>  DNA
<213>  Artificial sequence

<220>
<223>  Exemplary sequence of the oligonucleotide code II`

<220>
<221>  misc_feature
<222>  (9)..(9)
<223>  n is inosine

<220>
<221>  misc_feature
<222>  (10)..(10)
<223>  n is an abasic site

<220>
<221>  misc_feature
<222>  (11)..(11)
<223>  n is an abasic site

<220>
<221>  misc_feature
<222>  (12)..(12)
<223>  n is inosine

<220>
<221>  misc_feature
<222>  (13)..(13)
<223>  n is inosine

<220>
<221>  misc_feature
<222>  (14)..(14)
<223>  n is an abasic site

<220>
<221>  misc_feature
<222>  (15)..(15)
<223>  n is an abasic site

<220>
<221>  misc_feature
<222>  (16)..(16)
<223>  n is inosine

<400>  23
atacaggtnn nnnnnnagag gact                                              24

<210>  24
<211>  16
<212>  DNA
<213>  Artificial sequence

<220>
<223>  Exemplary sequence of the oligonucleotide code II

<220>
<221>  misc_feature
<222>  (9)..(9)
<223>  n is 7-deaza-8-azaguanosine

<220>
<221>  misc_feature
<222>  (10)..(10)
<223>  n is 7-deaza-8-azaadenosine

<400>  24
ctctcatgnn tcacct                                                    16


<210>  25
<211>  16
<212>  DNA
<213>  Artificial sequence

<220>
<223>  Exemplary sequence of the oligonucleotide code II


<220>
<221>  misc_feature
<222>  (8)..(9)
<223>  each n is 7-deaza-8-azaguanosine

<400>  25
ctctcatnna tcacct                                                    16


<210>  26
<211>  16
<212>  DNA
<213>  Artificial sequence

<220>
<223>  Exemplary sequence of the oligonucleotide code II


<220>
<221>  misc_feature
<222>  (6)..(6)
<223>  n is 7-deaza-8-azaadenosine

<220>
<221>  misc_feature
<222>  (10)..(10)
<223>  n is 7-deaza-8-azaadenosine

<400>  26
ctctcntggn tcacct                                                    16